# EUROPEAN PATENT APPLICATION

(11) **EP 4 534 151 A1**
(43) Date of publication of application: **09.04.2025**
(21) Application number: 23815950.3
(22) Date of filing: 26.05.2023
(51) Int. Cl.: A61Q 5/12, A61K 8/06, A61K 8/34, A61K 8/365, A61K 8/41, A61K 8/73, A61K 8/81, A61K 8/86

(54) **PRODUCTION METHOD FOR AQUEOUS COMPOSITION**

(30) Priority: 30.05.2022 JP 2022087748; 23.03.2023 JP 2023046568
(71) Applicant: Kao Corporation, Tokyo 103-8210 (JP)
(72) Inventor: MIYOSHI Eisuke, Tokyo 131-8501 (JP); KAWAMOTO Koichi, Tokyo 131-8501 (JP); SARUKAWA Yuri, Tokyo 131-8501 (JP); MIENO Akiko, Tokyo 131-8501 (JP); FURUTA Saya, Tokyo 131-8501 (JP)
(74) Representative: Hoffmann Eitle
(86) International application number: PCT/JP2023/019642
(87) International publication number: WO 2023/234193

(57) **Abstract**

A method for producing an aqueous composition, the method including steps 1 to 3 described below in this order: step 1: preparing a mixture 1 containing (B) an anionic polymer, (C) a nonionic polymer, and water; step 2: mixing the mixture 1 with (D) an acid or an aqueous solution thereof to prepare a mixture 2 containing the anionic polymer (B), the nonionic polymer (C), the acid (D), and water; and step 3: mixing the mixture 2 with (A) a cationic polymer.

## Description

### Technical Field

The present invention relates to a method for producing an aqueous composition and an emulsified composition.

### Background Art

Cosmetic compositions containing cationic polymers and anionic polymers are known. For example, JP S53-139734 A (PTL 1) describes that using a composition for treating a keratin material, which is characterized by containing at least one anionic polymer and at least one cationic polymer in a solvent medium, makes it possible to fix the anionic polymer to a keratin material that can particularly include hair, skin, or nails. It is assumed that a complex formed by an interaction between the anionic polymer and the cationic polymer is related to the reason for the above-described effect.

As a composition containing a complex formed from a cationic polymer and an anionic polymer (hereinafter also referred to as "polyion complex"), a composition in the form of an oil-in-water type or water-in-oil type emulsified composition is known. In general, improvement of stability over time is a problem in emulsified compositions, and stable emulsified compositions or methods for producing stable emulsified compositions have also been studied.

For example, JP H11-116445 (PTL 2) discloses that a hair treatment composition in a stable emulsified state containing a polymer supplied in a powder form can be easily produced by producing a hair treatment composition through a step of emulsifying an oil content and a water content with a surfactant, a step of dissolving a powdery cationic polymer and a powdery anionic polymer in water, and a step of mixing them.

JP 2020-536857 (PTL 3) discloses that a cosmetic composition such as a cleansing care product including a predetermined polymerization product obtained by reacting a system including an anionic polymer, an amphoteric polymer, and water, an optional cationic polymer, an optional active ingredient, an optional additive, and an optional cosmetically acceptable medium can leave an effective amount of the active ingredient on hair after the hair is rinsed, thereby making the hair easy to comb, smooth, soft, moist, and nourished while the other ingredients in the cleansing care product other than the active ingredient are easy to wash off. Also disclosed is a method for producing the composition including a step of mixing and reacting a system including the anionic polymer, the amphoteric polymer, and water to obtain the polymerization product, and a step of mixing the polymerization product, an optional cationic polymer, an optional active ingredient, an optional additive, and an optional cosmetically acceptable medium to obtain the composition.

JP 2021-31468 (PTL 4) discloses that a cosmetic composition for keratin fibers including at least one ionic polymer selected from the group consisting of cationic polymers, anionic polymers, amphoteric polymers, and mixtures thereof, at least one polyion complex including a predetermined crosslinking agent, at least one texturing agent such as a hydrophilic thickener, and water can provide improved texture such as smoothness, softness, and less stickiness to keratin fibers such as hair. In Examples, a method is described in which an aqueous solution of a cationic polymer and an acidic compound are mixed to prepare a gel solution of a polyion complex, and a nonionic polymer is added thereto and homogenized to obtain a uniform composition in the form of emulsion.

### Summary of Invention

The present invention relates to the following.
[1] A method for producing an aqueous composition,
   the aqueous composition comprising (A) a cationic polymer, (B) an anionic polymer, (C) a nonionic polymer, (D) an acid, and water,
   the method including steps 1 to 3 described below in this order:
      step 1: preparing a mixture 1 comprising the anionic polymer (B), the nonionic polymer (C), and water;
      step 2: mixing the mixture 1 with the acid (D) or an aqueous solution thereof to prepare a mixture 2 comprising the anionic polymer (B), the nonionic polymer (C), the acid (D), and water; and
      step 3: mixing the mixture 2 with the cationic polymer (A).
[2] A method for producing an emulsified composition,
   the emulsified composition comprising (A) a cationic polymer, (B) an anionic polymer, (C) a nonionic polymer, (D) an acid, an oily component, and water,
   the method including steps 1 to 3 and step 4-1 or 4-2 described below in this order:
      step 1: preparing a mixture 1 comprising the anionic polymer (B), the nonionic polymer (C), and water;
      step 2: mixing the mixture 1 with the acid (D) or an aqueous solution thereof to prepare a mixture 2 comprising the anionic polymer (B), the nonionic polymer (C), the acid (D), and water;
      step 3: mixing the mixture 2 with the cationic polymer (A) to prepare a mixture 3 comprising the cationic polymer (A), the anionic polymer (B), the nonionic polymer (C), the acid (D), and water;
      step 4-1: mixing an aqueous phase containing water with an oily phase containing an oily component to prepare an emulsified product, and then mixing the emulsified product with the mixture 3; and
      step 4-2: mixing the mixture 3 with an aqueous phase containing water, and then mixing the mixture with an oily phase containing an oily component for emulsification.

### Description of Embodiments

As described above, a composition containing a polyion complex and a method for producing the same have been studied. However, there is still room for improvement in performance when a composition containing a polyion complex is used as a cosmetic composition or a fiber treatment composition for head decoration products.

For example, in a hair conditioner which is a type of hair cosmetic composition, it is required that the texture of hair after the hair conditioner is applied is good, entanglement of the hair is suppressed, and the hair after treatment does not spread even under high humidity conditions, and the hair has excellent moisture resistance. Further, the hair treatment effect may be lost by one time of hair washing, and the durability (washing resistance) of the treatment effect has been desired.

As a method for improving the texture of hair when the hair conditioner is applied to hair or after hair is treated, a method is generally known in which a cationic surfactant, a higher alcohol, or the like is added to the hair conditioner, but there is a problem that an emulsified composition obtained by blending these components to a composition containing a polyion complex is low in stability.

The present invention relates to a method for producing an aqueous composition and an emulsified composition that contain a polyion complex, has high stability, can improve the texture improving effect of an object to be treated, the durability of the effect, and the moisture resistance, can improve the effect of suppressing entanglement of hair or fibers for head decoration products and the durability thereof particularly when the compositions are used for treating hair or fibers for head decoration products, and can suppress the spread of hair or fibers for head decoration products after treatment under high humidity conditions.

The inventors of the present invention have found that the above-described problems can be solved by producing an aqueous composition containing a cationic polymer, an anionic polymer, a nonionic polymer, an acid, and water through a predetermined process, and by producing an emulsified composition using the aqueous composition.

The present invention can provide a method for producing an aqueous composition and an emulsified composition that contain a polyion complex, has high stability, can improve the texture improving effect of an object to be treated, the durability of the effect, and the moisture resistance, can improve the effect of suppressing entanglement of hair or fibers for head decoration products and the durability thereof particularly when the compositions are used for treating hair or fibers for head decoration products, and can suppress the spread of hair or fibers for head decoration products after treatment under high humidity conditions.

### [Method for Producing Aqueous Composition and Emulsified Composition]

The present invention relates to a method for producing an aqueous composition,
the aqueous composition containing (A) a cationic polymer, (B) an anionic polymer, (C) a nonionic polymer, (D) an acid, and water,
the method including steps 1 to 3 described below in this order:
   step 1: preparing a mixture 1 containing the anionic polymer (B), the nonionic polymer (C), and water;
   step 2: mixing the mixture 1 with the acid (D) or an aqueous solution thereof to prepare a mixture 2 containing the anionic polymer (B), the nonionic polymer (C), the acid (D), and water; and
   step 3: mixing the mixture 2 with the cationic polymer (A).

The present invention also relates to a method for producing an emulsified composition,
the emulsified composition containing (A) a cationic polymer, (B) an anionic polymer, (C) a nonionic polymer, (D) an acid, an oily component, and water,
the method including steps 1 to 3 and step 4-1 or 4-2 described below in this order:
   step 1: preparing a mixture 1 containing the anionic polymer (B), the nonionic polymer (C), and water;
   step 2: mixing the mixture 1 with the acid (D) or an aqueous solution thereof to prepare a mixture 2 containing the anionic polymer (B), the nonionic polymer(C), the acid (D), and water;
   step 3: mixing the mixture 2 with the cationic polymer (A) to prepare a mixture 3 containing the cationic polymer (A), the anionic polymer (B), the nonionic polymer (C), the acid (D), and water;
   step 4-1: mixing an aqueous phase containing water with an oily phase containing an oily component to prepare an emulsified product, and then mixing the emulsified product with the mixture 3; and
   step 4-2: mixing the mixture 3 with an aqueous phase containing water, and then mixing the mixture with an oily phase containing an oily component for emulsification.

### <Definitions>

Hereinafter, the aqueous composition and the emulsified composition are collectively referred to as "composition", and the production method is collectively referred to as "production method of the present invention".

In the present specification, "aqueous phase" means a component constituting a water phase in the production process of an emulsified composition, and "oily phase" means a component constituting an oil phase in the production process of an emulsified composition.

The production method of the present invention can provide an aqueous composition and an emulsified composition that contain a polyion complex, has high stability, can improve the texture improving effect of an object to be treated, the durability of the effect, and the moisture resistance, can improve the effect of suppressing entanglement of hair or fibers for head decoration products and the durability thereof particularly when the compositions are used for treating hair or fibers for head decoration products, and can suppress the spread of hair or fibers for head decoration products after treatment under high humidity conditions. Hereinafter, steps 4-1 and 4-2 may be collectively referred to as "step 4".

The reason for the production method of the present invention exhibiting the above-described effects is not clear, but it is considered as follows.

The composition produced through the present invention contains a polyion complex formed from the component (A) and the component (B). In the polyion complex, a crosslinked structure is formed by an ionic interaction between the component (A) and the component (B), and it is assumed that this structure causes the film obtained by using the composition of the present invention to exhibit a high elastic modulus.

Further, the composition produced through the present invention contains a nonionic polymer as the component (C). It is considered that in the film obtained by using the composition, the rigid crosslinked structure of the polyion complex formed by the component (A) and the component (B) is reinforced by complexing with the long-chain and flexible non-crosslinked structure of the component (C). Since a hydrogen bond can be formed between the component (B) and the component (C), it is considered that the component (C) is held in the crosslinked structure by the component (B) to form a network having higher strength in the film. Accordingly, it is considered that when the composition is used, the effect of improving the texture and the moisture resistance of the object to be treated can be obtained with a single treatment, and the treatment effect is not lost even when washing is performed thereafter, and the durability of the effect can be obtained.

In the production method of the present invention, first, in step 1, a mixture 1 containing (B) an anionic polymer, (C) a nonionic polymer, and water is prepared. Subsequently, the mixture 1 is mixed with (D) an acid or an aqueous solution thereof (step 2), and finally mixed with (A) a cationic polymer (step 3). By performing the step 1 first, the component (B) and the component (C) form a hydrogen bond, and the component (C) is easily immobilized in the crosslinked structure of the polyion complex because of the component (B). It is considered that the component (C) is thus not washed away and is likely to remain in the film even when washing is performed after the film formation, and the durability of the treatment effect improves.

The network gel containing a highly stable polyion complex is formed by mixing the mixture 1 with the acid (D) or an aqueous solution thereof in the step 2, and then mixing the cationic polymer (A) in the step 3. Thus, even when the oily phase is blended to form an emulsified product in the step 4 in the production of the emulsified composition, the network gel is dispersed in a good state without causing aggregation, separation, or the like, and thus it is considered that the stability of the emulsified composition improves.

When the aqueous composition and the emulsified composition are used for treating hair or fibers for head decoration products, it is considered that the network gel dispersed in a good state uniformly adheres to hair to improve the effect of suppressing entanglement of hair or fibers for head decoration products and the durability thereof, and to suppress spreading of treated hair or fibers for head decoration products under high humidity conditions.

The composition produced through the present invention is preferably a cosmetic composition for keratin materials such as skin, hair, eyelashes, eyebrows, and nails, or a fiber treatment composition for head decoration products, more preferably a cosmetic composition for skin or hair, and a fiber treatment composition for head decoration products, even more preferably a cosmetic composition for skin or hair, and even more preferably a hair cosmetic composition.

Among the cosmetic compositions, examples of the product form of the skin cosmetic composition include a body soap, a facial cleanser, a bath agent, a deodorant preparation, a makeup cosmetic, a sunscreen, a makeup foundation, a milky lotion, a beauty essence, and a cream.

Examples of the product form of the hair cosmetic composition include a hair shampoo, a hair rinse, a hair conditioner, a hair treatment (including a leave-on type), a hair styling agent, a hair coloring agent, and a permanent agent. Among these, from the viewpoint of the effectiveness of the effects of the present invention, a hair conditioner, a hair treatment, or a hair styling agent is preferable.

The fibers for head decoration products may be either naturally derived fibers or synthetic fibers, but naturally derived fibers are preferred. The naturally derived fibers refer to fibers collected from a natural plant or animal, or fibers artificially produced using collagen, casein, soybean, peanut, corn, silk waste silk fibroin, or the like as a raw material and used for a head decoration product. Among these, fibers artificially produced from collagen, casein, soybean, peanut, corn, silk waste silk fibroin, or the like as a raw material are preferable, regenerated protein fibers such as regenerated collagen fibers using collagen as a raw material and regenerated silk fibers using silk fibroin as a raw material are more preferable, and regenerated collagen fibers are even more preferable.

Regenerated collagen fibers can be produced by known techniques. The composition of the regenerated collagen fibers does not need to be 100% collagen, and the composition may contain natural polymers, synthetic polymers, additives, and the like for quality improvement. Further, the regenerated collagen fibers may be post-processed.

The regenerated collagen fibers are preferably in the form of filaments. The filaments are generally taken from a bobbin wound or boxed condition. It is also possible to directly utilize the filaments coming out of the drying step in the production process of regenerated collagen fibers.

Examples of the synthetic fiber include a fiber containing a synthetic resin as a main component. The synthetic resin is preferably a thermoplastic resin, and more preferably one or more selected from the group consisting of polyester resins, polyamide resins, polyimide resins, polyamide-imide resins, vinyl chloride resins, polycarbonate resins, polyphenylene sulfide resins, and modacrylic resins (copolymer of acrylonitrile and vinyl chloride), from the viewpoint of ease of production of the synthetic fiber and from the viewpoint of obtaining a feeling close to that of hair. The term "main component" as used herein means a component whose content in the synthetic fiber is preferably 50 mass% or more, more preferably 60 mass% or more, even more preferably 70 mass% or more, even more preferably 80 mass% or more, and even more preferably 90 mass% or more, and is 100 mass% or less.

The synthetic fiber may further contain, in addition to the above-described synthetic resin, various additives such as a flame retardant, a flame retardant aid, a light or heat stabilizer, a fluorescent agent, an antioxidant, an antistatic agent, and an ultraviolet absorber, as long as the effects of the present invention are not impaired.

The emulsified composition produced through the present invention is preferably an oil-in-water emulsified composition from the viewpoint of stability and from the viewpoint of improvement in feeling upon use.

Hereinafter, each component to be blended in the aqueous composition and the emulsified composition will be described.

### (Component (A): Cationic Polymer)

The cationic polymer (A) is a polymer capable of forming a polyion complex by interaction with the anionic polymer (B).

The cationic polymer as the component (A) is preferably a polymer having a cationic group and being positively charged as a total charge. The component (A) may have an anionic group, a nonionic group, or an amphoteric group such as a betaine group in addition to the cationic group as long as the effects of the present invention are not impaired.

In the present specification, the cationic group is a cationic group or a group that can be ionized to become a cationic group, and specific examples thereof include a primary amino group, a secondary amino group, a tertiary amino group, and a quaternary ammonium group.

The anionic group is an anionic group or a group that can be ionized to become an anionic group, and specific examples thereof include one or more selected from the group consisting of acidic groups such as a carboxyl group, a sulfonic acid group, and a phosphoric acid group, preferably one or more selected from a carboxyl group and a sulfonic acid group, and more preferably a carboxyl group. At least a part of the anionic groups may be neutralized to form a salt.

The component (A) is preferably a cationic polymer having a viscosity when in a 1 mass% aqueous solution form at 30°C of 1000 mPa·s or more. It is considered that using the component (A) having the viscosity of 1000 mPa·s or more improves the lubricity of the obtained film, which further improves the texture of the object to be treated, and when it is used for treating hair or fibers for head decoration products, the effect of suppressing entanglement of hair or fibers for head decoration products improves. In addition, it is considered that the structure of the polyion complex is hardly destroyed and the stability is improved even when an oily component is added to form an emulsified composition, since the polyion complex containing the component (A) has high stability.

The viscosity of a 1 mass% aqueous solution of the component (A) at 30°C is more preferably 1300 mPa·s or more, even more preferably 1500 mPa·s or more, even more preferably 1700 mPa·s or more, and even more preferably 1800 mPa·s or more, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition. The upper limit of the viscosity is not particularly limited, but is preferably 100000 mPa·s or less, more preferably 80000 mPa·s or less, even more preferably 50000 mPa·s or less, even more preferably 30000 mPa·s or less, and even more preferably 25000 mPa·s or less, from the viewpoint of ease of forming a polyion complex and handleability. The viscosity of a 1 mass% aqueous solution of the component (A) at 30°C is preferably 1000 mPa·s or more, more preferably 1000 mPa·s or more and 100000 mPa·s or less, even more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.

The cationic charge density of the component (A) is preferably 0.1 mmol/g or more, more preferably 0.2 mmol/g or more, even more preferably 0.3 mmol/g or more, and even more preferably 0.5 mmol/g or more, from the viewpoint of facilitating formation of a polyion complex through interaction with the component (B), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of the improvement of the stability of the composition. The cationic charge density is preferably 20 mmol/g or less, more preferably 15 mmol/g or less, even more preferably 12 mmol/g or less, even more preferably 10 mmol/g or less, even more preferably 7.0 mmol/g or less, and even more preferably 5.0 mmol/g or less from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition. The cationic charge density of the component (A) is preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.

The cationic charge density of the component (A) is the number of moles of cationic groups contained per polymer 1 g. When at least a part of the cationic groups of the component (A) is in the form of a neutralized salt, the number of moles of the cationic groups includes the number of moles of the cationic groups in the form of a salt.

Two or more polymers may be used as the component (A), and in this case, the cationic charge density of the component (A) is determined by calculating a weight from the cationic charge density and the blending amount of each polymer.

As the component (A), any of a natural polymer such as a cationized polysaccharide or a cationized product thereof and a synthetic polymer can be used, but from the viewpoint of availability, a synthetic polymer is preferable.

Specific examples of the polymer used as the component (A) preferably include cationized polyvinyl alcohols, polyethyleneimines, methacryloylethyltrimethylammonium salt polymers, quaternized dialkylaminoalkyl (meth)acrylate polymers, diallylquaternized ammonium salts, methacrylamidopropyltrimethylammonium salt polymers, vinylimidazoliumtrichloride-vinylpyrrolidone copolymers (polyquaternium-16), vinylpyrrolidone-alkylamino (meth)acrylate copolymers, vinylpyrrolidone-alkylamino (meth)acrylate-vinylcaprolactam copolymers, alkylacrylamide-(meth)acrylate-alkylaminoalkylacrylamide-polyethylene glycol (meth)acrylate copolymers, and adipic acid-dimethylaminohydroxypropylethylenetriamine copolymers having a viscosity when in a 1 mass% aqueous solution form at 30°C of 1000 mPa·s or more, and one or two or more of these can be used. In the present specification, "(meth)acrylic acid" means acrylic acid or methacrylic acid, and "(meth)acrylate" means acrylate or methacrylate.

Among these, examples of the methacryloylethyltrimethylammonium salt polymer include a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), and a methacryloylethyldimethylbetaine-methacryloylethyltrimethylammonium chloride-2-hydroxyethyl methacrylate copolymer (polyquaternium-48).

Examples of the quaternized dialkylaminoalkyl (meth)acrylate polymer include a vinylpyrrolidone-N,N-dimethylaminoethyl methacrylate diethyl sulfate copolymer (polyquaternium-11) and an N,N-dimethylaminoethyl methacrylate diethyl sulfate-N,N-dimethylacrylamide-polyethylene glycol dimethacrylate copolymer (polyquaternium-52).

Examples of the diallyl quaternized ammonium salt polymer include a dimethyldiallylammonium chloride polymer (polyquaternium-6), a dimethyldiallylammonium chloride-acrylic acid copolymer (polyquaternium-22), a dimethyldiallylammonium chloride-acrylamide copolymer (polyquaternium-7), and an acrylamide-acrylic acid-dimethyldiallylammonium chloride copolymer (polyquaternium-39).

Examples of the methacrylamidopropyltrimethylammonium salt polymer include a methacrylamidopropyltrimethylammonium chloride polymer, a vinylpyrrolidone-methacrylamidopropyltrimethylammonium chloride copolymer, an acrylic acid-methyl acrylate-methacrylamidopropyltrimethylammonium chloride copolymer (polyquaternium-47), and an acrylic acid-acrylamide-methacrylamidopropyltrimethylammonium chloride copolymer (polyquaternium-53).

The component (A) is preferably a polymer containing a constituent unit represented by the following General Formula (1) from the viewpoint of the improvement of the film-forming ability, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. 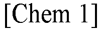 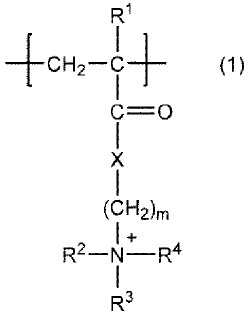 wherein R¹ is a hydrogen atom or a methyl group, and R² to R⁴ are each independently an alkyl group having 1 or more and 4 or less carbons, X is -O- or -NH-, and m is the number of 1 to 4.

In Formula (1), R¹ is preferably a methyl group, and R² to R⁴ are each independently preferably an alkyl group having 1 or more and 3 or less carbons, more preferably a methyl group or an ethyl group, and even more preferably a methyl group.

X in Formula (1) is preferably -O-, and m is preferably 1 or more and 3 or less, and more preferably 2 or more and 3 or less.

Examples of the constituent unit represented by Formula (1) include one or more selected from the group consisting of a constituent unit derived from a methacryloylethyltrimethylammonium salt, a constituent unit derived from N,N-dimethylaminoethylmethacrylic acid diethyl sulfate, and a constituent unit derived from a methacrylamidopropyltrimethylammonium salt, and preferably one or more selected from the group consisting of a constituent unit derived from a methacryloylethyltrimethylammonium salt and a constituent unit derived from N,N-dimethylaminoethylmethacrylic acid diethyl sulfate.

The component (A) may be a polymer containing an additional constituent unit other than the constituent unit represented by Formula (1). Examples of the additional constituent unit include constituent units derived from vinyl monomers such as vinylpyrrolidone, amphoteric monomers such as (meth)acryloylethyldimethylbetaine, hydroxyalkyl (meth)acrylates such as (meth)acrylic acid alkyl esters and 2-hydroxyethyl (meth)acrylate, and acrylamides such as N,N-dimethylacrylamide. The component (A) may also be a crosspolymer crosslinked with di(meth)acrylic acid polyethylene glycol or the like.

In the component (A), the content of the constituent unit represented by Formula (1) is preferably 8.0 mol% or more, more preferably 10 mol% or more, and is 100 mol% or less, based on all constituent units constituting the component (A), from the viewpoint of improving the film-forming ability, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof.

The component (A) may be used alone or in combination of two or more thereof. Among the above, the component (A) is preferably one or more selected from the group consisting of a methacryloyl ethyltrimethylammonium salt polymer and a quaternary dialkylaminoalkyl (meth)acrylate salt polymer, more preferably one or more selected from the group consisting of a methacryloyl ethyltrimethylammonium chloride polymer (polyquaternium-37), a methacryloyl ethyldimethylbetaine-methacryloyl trimethylammonium chloride-methacrylic acid methoxy polyethylene glycol copolymer (polyquaternium-49), a methacryloyl ethyl dimethyl betaine-methacryloyl ethyl trimethyl ammonium chloride-2-hydroxyethyl methacrylate copolymer (polyquaternium-48) and an N,N-dimethylaminoethyl methacrylic acid diethyl sulfate-N,N-dimethylacrylamide-polyethylene glycol dimethacrylate copolymer (polyquaternium-52), and even more preferably one or more selected from the group consisting of a methacryloyl ethyl trimethyl ammonium chloride polymer (polyquaternium-37) and an N,N-dimethylaminoethyl methacrylic acid diethyl sulfate-N,N-dimethylacrylamide-polyethylene glycol dimethacrylate copolymer (polyquaternium-52), from the viewpoint of easily setting the viscosity when in a 1 mass% aqueous solution form at 30°C to a value equal to or larger than a predetermined value, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability of thereof, from the viewpoint of the improvement of the moisture resistance, and from the viewpoint of the improvement of the stability of the composition.

As the component (A), a commercially available polymer can also be used. Specific examples thereof include "Cosmedia Ultragel 300" (polyquaternium-37, cationic charge density: 4.81 mmol/g) manufactured by BASF Japan Ltd., "SOFCARE KG-101W-E" (polyquaternium-52, cationic charge density: 0.83 mmol/g), and "SOFCARE KG-301W" (polyquaternium-52, cationic charge density: 1.84 mmol/g) manufactured by Kao Corporation.

### (Component (B): Anionic Polymer)

The component (B) is a polymer capable of forming a polyion complex through interaction with the component (A). **In** the present specification, "anionic polymer" means a polymer having an anionic group and substantially having no cationic group and no amphoteric group. "Having substantially no cationic group and no amphoteric group" means that the molar amount of the cationic group and the amphoteric group relative to the anionic group is preferably 0.1% or less.

The anionic group of the component (B) is preferably an acidic group such as a carboxy group, a sulfonic acid group, or a phosphoric acid group, and is more preferably a carboxy group from the viewpoint of facilitating formation of a polyion complex through interaction with the component (A) and from the viewpoint of availability. In the component (B), at least a part of the anionic groups may be neutralized to form a salt.

### <Component (B1): Crosslinked Polymer Containing (Meth)Acrylic Acid-Derived Constituent Unit, and Anionic Polysaccharides>

The component (B) preferably contains (B1) one or more selected from the group consisting of a cross-linked polymer containing a (meth)acrylic acid-derived constituent unit and an anionic polysaccharide, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition.

Among the components (B 1), examples of the cross-linked polymer containing a constituent unit derived from (meth)acrylic acid include homopolymer or a copolymer of (meth)acrylic acid having a crosslinked structure, and a salt thereof. The term "crosslinked structure" as used herein refers to a three-dimensional network structure in which polymer chains, which are the main structure of a polymer, are linked within a polymer chain or between polymer chains. The method for forming the cross-linked structure is not particularly limited, and examples thereof include a method of cross-linking simultaneously with polymerization such as polycondensation or radical polymerization, and a method of cross-linking polymer chains afterwards.

Examples of the homopolymer of (meth)acrylic acid include a polyacrylic acid, a polymethacrylic acid, and a salt thereof.

Examples of the copolymer of (meth)acrylic acid include a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these copolymers can be used.

Among these, as the copolymer of (meth)acrylic acid, a (meth)acrylic acid/(meth)acrylic acid alkyl ester is preferable, and an acrylic acid/acrylic acid alkyl ester is more preferable. Examples of the (meth)acrylic acid alkyl ester include (meth)acrylic acid alkyl esters in which the number of carbons of the alkyl is preferably 1 or more, more preferably 4 or more, and even more preferably 8 or more, and is preferably 40 or less, more preferably 36 or less, and even more preferably 32 or less.

Specific examples of the cross-linked polymer containing a constituent unit derived from (meth)acrylic acid include a carboxyvinyl polymer or a salt thereof, an (acrylates/alkyl acrylate (C10-30)) crosspolymer, a (Na acrylate/acryloyldimethyltaurine/dimethylacrylamide) crosspolymer, and an acrylates crosspolymer-4.

Among the components (B1), examples of the anionic polysaccharides include polysaccharides having a carboxyl group (hyaluronic acid, alginic acid, pectic acid, carboxymethyl cellulose, xanthan gum, and the like), sulfates of polysaccharides (carrageenan, keratan sulfate, dermatan sulfate, sulfated starch, heparin, heparan sulfate), and a salt thereof, and one or two or more of these can be used.

Among the above, the anionic polysaccharide is preferably a polysaccharide having a carboxyl group or a salt thereof, more preferably alginic acid, carboxymethyl cellulose, or a salt thereof, and even more preferably alginic acid or a salt thereof, from the viewpoint of the texture improving effect of the object to be treated and the improvement of the durability of the effect, in particular, the effect of suppressing the entanglement of hair or fibers for head decoration products when used for treating hair or fibers for head decoration products and the improvement of the durability thereof.

When the component (B1) is a salt, examples of the salt include alkali metal salts such as a sodium salt and a potassium salt, and alkaline earth metal salts, and from the viewpoint of availability, an alkali metal salt is preferable.

Among the above, the component (B 1) is preferably one or more selected from the group consisting of a carboxyvinyl polymer, an (acrylates/alkyl acrylate (C10-30)) cross polymer, and an anionic polysaccharide, more preferably an anionic polysaccharide or a salt thereof, and even more preferably alginic acid or a salt thereof, from the viewpoints of the texture improving effect of the object to be treated and the improvement of the durability of the effect, in particular, the effect of suppressing entanglement of hair or fibers for head decorative products when used for treating hair or fibers for head decorative products and the improvement of the durability thereof, from the viewpoint of the improvement of the moisture resistance, and from the viewpoint of the improvement of the stability of the composition.

The anionic charge density of the component (B1) is not particularly limited, but is preferably 1.0 mmol/g or more, more preferably 2.0 mmol/g or more, and even more preferably 3.5 mmol/g or more from the viewpoint of facilitating formation of a polyion complex through interaction with the component (A), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition. The charge density is preferably 25 mmol/g or less, more preferably 20 mmol/g or less, and even more preferably 15 mmol/g or less, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof. The anionic charge density of the component (B1) is preferably 1.0 mmol/g or more and 25 mmol/g or less, more preferably 2.0 mmol/g or more and 20 mmol/g or less, even more preferably 3.5 mmol/g or more and 15 mmol/g or less.

The anionic charge density of the component (B1) is the number of moles of anionic groups contained per polymer 1 g. When at least a part of the anion groups of the component (B1) is in the form of a neutralized salt, the number of moles of anion groups includes the number of moles of anion groups in the form of a salt.

Two or more polymers may be used as the component (B1), and in this case, the anionic charge density of the component (B1) is determined by calculating a weighted average from the anionic charge density and the blending amount of each polymer.

When the component (B1) is an anionic polysaccharide, the weight average molecular weight (Mw) thereof is preferably 2000 or more, more preferably 10000 or more, even more preferably more than 30000, and even more preferably 50000 or more from the viewpoint of improving the film-forming ability. The weight average molecular weight is preferably 3500000 or less, and more preferably 3000000 or less from the viewpoint of facilitating the formation of a polyion complex through the interaction with the component (A), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of moisture resistance, and from the viewpoint of the improvement of the stability of the composition. The weight average molecular weight (Mw) of the anionic polysaccharide as the component (B 1) is preferably 2000 or more and 3500000 or less, more preferably 10000 or more and 3000000 or less, even more preferably more than 30000 and 3000000 or less, and even more preferably 50000 or more and 3000000 or less.

The weight average molecular weight of the component (B1) can be measured by gel permeation chromatography (GPC).

The content of the component (B1) in the component (B) is preferably 50 mass% or more, more preferably 60 mass% or more, still more preferably 70 mass% or more, even still more preferably 80 mass% or more, and is 100 mass% or less from the viewpoint of improving the film-forming ability, from the viewpoint of easily forming a polyion complex through interaction with the component (A), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of improving the moisture resistance, and from the viewpoint of improving the stability of the composition.

### <Component (B2): Anionic Polymer Other Than Component (B1)>

The component (B) preferably further contains (B2) an anionic polymer other than the component (B 1) from the viewpoint of further improving the texture improving effect of the object to be treated and the durability of the effect, in particular, the effect of suppressing entanglement of hair or fibers for head decoration products when used in for treating hair or fibers for head decoration products and the improvement of the durability thereof, and from the viewpoint of the improvement of the moisture resistance.

It is considered that the component (B2) forms a hydrogen bond with the nonionic polymer (C), whereby the component (C) can be immobilized in the crosslinked structure of the polyion complex formed by the component (A)-the component (B). It is considered that due to this effect, even when rinsing or washing is performed after treating an object to be treated with the composition, the component (C) is not washed away and is likely to remain in the film, and the durability of the treatment effect is improved.

The molecular weight of the component (B2) is preferably 1000 or more, more preferably 2000 or more, even more preferably 5000 or more, even more preferably 10000 or more, and preferably 50000 or less, more preferably 40000 or less, even more preferably 30000 or less from the viewpoint of contributing to the immobilization of the component (C) to further improve the texture improving effect of the object to be treated and the durability of the effect, in particular, the effect of suppressing entanglement of hair or fibers for head decoration products when used for treating hair or fibers for head decoration products and the improvement of the durability thereof, and from the viewpoint of the improvement of the moisture resistance. The molecular weight of the component (B2) is preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.

The term "molecular weight" as used herein means a weight average molecular weight and can be measured by gel permeation chromatography (GPC).

The component (B2) is preferably a polymer having a carboxy group with a molecular weight of 1000 or more and 50000 or less, more preferably 1000 or more and 30000 or less, and more preferably a non-crosslinked polymer containing a constituent unit derived from (meth)acrylic acid with a molecular weight of 1000 or more and 30000 or less.

Examples of the non-crosslinked polymer containing a constituent unit derived from (meth)acrylic acid suitably used as the component (B2) include, in addition to poly(meth)acrylic acid, a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these can be used.

Examples of the polymer having a carboxy group other than those described above and suitably used as the component (B2) include polymaleic acid, a maleic acid/diisobutylene copolymer, a maleic acid/styrene copolymer, a benzoic acid formaldehyde condensate, a benzoic acid/phenol/formaldehyde condensate, and a salt thereof.

Among the above, the component (B2) is preferably one or more selected from the group consisting of a poly(meth)acrylic acid, a (meth)acrylic acid/(meth)acrylic acid alkyl ester copolymer, and a salt thereof from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and from the viewpoint of the improvement of the moisture resistance.

Examples of the poly(meth)acrylic acid or a salt thereof include polyacrylic acid, polymethacrylic acid, and a salt thereof, and polyacrylic acid or a salt thereof is preferable.

As the (meth)acrylic acid/(meth)acrylic acid alkyl ester copolymer or a salt thereof, an acrylic acid/acrylic acid alkyl ester or a salt thereof is more preferable. Examples of the (meth)acrylic acid alkyl ester include a (meth)acrylic acid alkyl ester in which the number of carbons in the alkyl is preferably 1 or more, more preferably 4 or more, even more preferably 8 or more, even more preferably 12 or more, and preferably 40 or less, more preferably 36 or less, even more preferably 32 or less, even more preferably 24 or less.

The (meth)acrylic acid/(meth)acrylic acid alkyl ester is preferably an acrylic acid/acrylic acid alkyl ester, more preferably one or more selected from the group consisting of an (acrylic acid/octyl acrylate) copolymer and an (acrylic acid/stearyl acrylate) copolymer, and even more preferably an (acrylic acid/stearyl acrylate) copolymer.

When the component (B2) is a salt, examples of the salt include alkali metal salts such as a sodium salt and a potassium salt, and alkaline earth metal salts, and from the viewpoint of availability, an alkali metal salt is preferable.

The anionic charge density of the component (B2) is not particularly limited, but is preferably 1.0 mmol/g or more, more preferably 3.0 mmol/g or more, and even more preferably 5.0 mmol/g or more, and is preferably 25 mmol/g or less, more preferably 20 mmol/g or less, and even more preferably 15 mmol/g or less from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and from the viewpoint of the improvement of the moisture resistance. The anionic charge density of the component (B2) is preferably 1.0 mmol/g or more and 25 mmol/g or less, more preferably 3.0 mmol/g or more and 20 mmol/g or less, even more preferably 5.0 mmol/g or more and 15 mmol/g or less.

Two or more polymers may be used as the component (B2), and in this case, the anionic charge density of the component (B2) is determined by calculating a weighted average from the anionic charge density and the blending amount of each polymer.

When the component (B) contains the component (B2), the content of the component (B2) in the component (B) is preferably 1 mass% or more, more preferably 2 mass% or more, even more preferably 3 mass% or more, and even more preferably 5 mass% or more, and the content is preferably 50 mass% or less, more preferably 40 mass% or less, even more preferably 30 mass% or less, and even more preferably 20 mass% or less, from the viewpoint of further improving the texture improving effect of the object to be treated and the durability of the effect, in particular, from the viewpoints of the effect of suppressing entanglement of hair or fibers for head decoration products when used for treating hair or fibers for head decoration products and the improvement of the durability of the effect, and from the viewpoint of the improvement of the moisture resistance. The content of the component (B2) in the component (B) is preferably 1 mass% or more and 50 mass% or less, more preferably 2 mass% or more and 40 mass% or less, even more preferably 3 mass% or more and 30 mass% or less, and even more preferably 5 mass% or more and 20 mass% or less.

When the component (B) contains the component (B1) and the component (B2), the mass ratio of the component (B2) to the total amount of the component (B1) and the component (B2) [(B2)/{(B1) + (B2)}] is preferably 0.01 or more, more preferably 0.02 or more, even more preferably 0.05 or more, and even more preferably 0.08 or more from the viewpoint of further improving the texture improving effect of the object to be treated and the durability of the effect, in particular, from the viewpoints of the effect of suppressing entanglement of hair or fibers for head decoration products when used for treating hair or fibers for head decoration products and the improvement of the durability thereof, and from the viewpoint of the improvement of the moisture resistance. From the viewpoint of easily forming a polyion complex with the component (A), the mass ratio is preferably 1.0 or less, more preferably 0.80 or less, even more preferably 0.50 or less, and even more preferably 0.30 or less. The mass ratio [(B2)/{(B1) + (B2)}] is preferably 0.01 or more and 1.0 or less, more preferably 0.02 or more and 0.80 or less, even more preferably 0.05 or more and 0.50 or less, and even more preferably 0.08 or more and 0.30 or less.

### (Component (C): Nonionic Polymer)

In the present specification, the "nonionic polymer" means a polymer having substantially no cationic group, anionic group, or amphoteric group, which are ionic groups.

Examples of the component (C) include polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam, and one or two or more thereof can be used.

Examples of the polyalkylene glycol include homopolymers and copolymers of alkylene glycols in which the number of carbons of alkylene is 2 or more and 6 or less, preferably 2 or more and 4 or less, and more preferably 2 or more and 3 or less. Specific examples thereof include polyethylene glycol, polypropylene glycol, polytrimethylene ether glycol, polytetramethylene ether glycol, a polyethylene glycol-polypropylene glycol copolymer (polyoxyethylene polyoxypropylene glycol), a polyethylene glycol-polytrimethylene ether glycol copolymer, a polyethylene glycol-polytetramethylene ether glycol copolymer, and a polypropylene glycol-polytetramethylene ether glycol copolymer. Among these, one or more selected from the group consisting of polyethylene glycol, polypropylene glycol, and a polyethylene glycol-polypropylene glycol copolymer is preferable, and one or more selected from the group consisting of polyethylene glycol and a polyethylene glycol-polypropylene glycol copolymer is more preferable, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of availability.

Examples of the polyalkylene glycol derivative include terminal-modified products of the above-described polyalkylene glycols, such as polyalkylene glycol monoalkyl ethers, polyalkylene glycol dialkyl ethers, polyalkylene glycol monoaryl ethers, polyalkylene glycol diaryl ethers, polyalkylene glycol monofatty acid esters, polyalkylene glycol difatty acid esters, and polyalkylene glycol diglycidyl ethers. Among these, one or more selected from the group consisting of a polyalkylene glycol monoalkyl ether, a polyalkylene glycol dialkyl ether, a polyalkylene glycol monofatty acid ester, and a polyalkylene glycol difatty acid ester is preferable, and one or more selected from the group consisting of a polyalkylene glycol monofatty acid ester and a polyalkylene glycol difatty acid ester is more preferable, from the viewpoints of the texture improving effect of the object to be treated and the improvement of the durability of the effect, in particular, effect of suppressing the entanglement of hair or fibers for head decoration products when used for treating hair or fibers for head decoration products and the improvement of the durability thereof, from the viewpoint of the improvement of moisture resistance, and from the viewpoint of availability.

The number of carbons of the alkyl group in the mono- or dialkyl ether of polyalkylene glycol and the number of carbons of the fatty acid in the mono- or di-fatty acid ester of polyalkylene glycol are preferably 1 or more and 36 or less, more preferably 4 or more and 32 or less, even more preferably 8 or more and 24 or less, and even more preferably 12 or more and 22 or less. The number of carbons of the aryl group in the mono- or diaryl ether of polyalkylene glycol is preferably 6 or more and 36 or less, more preferably 6 or more and 32 or less, even more preferably 6 or more and 24 or less, and even more preferably 6 or more and 22 or less.

Specific examples of the polyalkylene glycol derivative include polyethylene glycol monomethyl ether, polyethylene glycol dimethyl ether, polyethylene glycol monobutyl ether, polyethylene glycol dibutyl ether, polyethylene glycol monooctyl ether, polyethylene glycol monodecyl ether, polyethylene glycol monododecyl ether, polyethylene glycol monotridecyl ether, polyethylene glycol-bisphenol A ether, polyethylene glycol dilaurate, polyethylene glycol distearate, polyethylene glycol diglycidyl ether, polyethylene glycol-polypropylene glycol-monomethyl ether, polyethylene glycol-polypropylene glycol-dimethyl ether, polyethylene glycol-polypropylene glycol-monobutyl ether, polyethylene glycol-polypropylene glycol-dibutyl ether, polyethylene glycol-polypropylene glycol-monooctyl ether, polyethylene glycol-polypropylene glycol-monodecyl ether, polyethylene glycol-polypropylene glycol-monododecyl ether, polyethylene glycol-polypropylene glycol-monotridecyl ether, polyethylene glycol-polypropylene glycol-bisphenol A ether, polyethylene glycol-polypropylene glycol-dilaurate, polyethylene glycol-polypropylene glycol distearate, and polyethylene glycol-polypropylene glycol-diglycidyl ether, and one or two or more of them can be used.

Examples of the polymer containing a constituent unit derived from vinylpyrrolidone include a homopolymer of vinylpyrrolidone and a copolymer of vinylpyrrolidone and a nonionic monomer. Specific examples thereof include polyvinylpyrrolidone, a vinylpyrrolidone/vinyl acetate copolymer, and a vinylpyrrolidone/methacrylamide/vinylimidazole copolymer.

Examples of the polymer containing a constituent unit derived from a (meth)acrylic acid ester include a homopolymer of a (meth)acrylic acid ester, and a copolymer of a (meth)acrylic acid ester and a nonionic monomer such as (meth)acrylamide, dimethylacrylamide, vinyl acetate, or vinyl methyl ether. Examples of the (meth)acrylic acid ester include (meth)acrylic acid hydroxyalkyl ester and (meth)acrylic acid alkyl ether in addition to the (meth)acrylic acid alkyl ester described above.

Specific examples of the polymer containing a constituent unit derived from a (meth)acrylic acid ester include a (dimethylacrylamide/hydroxyethyl acrylate/methoxyethyl acrylate) copolymer and a (hydroxyethyl acrylate/methoxyethyl acrylate) copolymer.

Among the above, the component (C) is preferably one or more selected from the group consisting of polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or di-fatty acid ester thereof, and polyvinylpyrrolidone, and even more preferably one or more selected from the group consisting of polyethylene glycol, a polyethylene glycol-polypropylene glycol copolymer, a mono- or di-fatty acid ester thereof, and polyvinylpyrrolidone, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance and from the viewpoint of the availability.

The weight average molecular weight (Mw) of the component (C) is preferably 2000 or more, more preferably 5000 or more, even more preferably 8000 or more, and preferably 2000000 or less, more preferably 1500000 or less from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability, from the viewpoint of the improvement of the moisture resistance, and from the viewpoint of the availability. The weight average molecular weight of the component (C) is preferably 2000 or more and 2000000 or less, more preferably 5000 or more and 1500000 or less, and even more preferably 8000 or more and 1500000 or less.

The weight average molecular weight of the component (C) can be measured by gel permeation chromatography (GPC).

### (Component (D): Acid)

The acid (D) contributes to stable formation of the polyion complex.

An organic acid or an inorganic acid can be used as the component (D), and an organic acid is preferable from the viewpoint of using the composition to be produced as a cosmetic composition. Examples of the organic acid include carboxylic acid-based compound and sulfonic acid-based compound other than the component (B), and the organic acid is preferably a compound having a molecular weight of 500 or less, more preferably 200 or less.

Examples of the carboxylic acid-based compound include aliphatic monocarboxylic acids having 4 or less carbons, such as acetic acid, propionic acid, and butanoic acid; aromatic monocarboxylic acids such as benzoic acid; aliphatic dicarboxylic acids such as malonic acid, succinic acid, glutaric acid, adipic acid, maleic acid, and fumaric acid; aromatic dicarboxylic acids such as phthalic acid and isophthalic acid; polycarboxylic acids such as polyglutamic acid; hydroxycarboxylic acids such as lactic acid, malic acid, glycolic acid, hydroxyacrylic acid, glyceric acid, tartaric acid, and citric acid; and acidic amino acids such as glutamic acid and aspartic acid, and one or two or more of these can be used.

Examples of the sulfonic acid-based compound include aliphatic sulfonic acids such as methanesulfonic acid and ethanesulfonic acid; aromatic sulfonic acids such as p-toluene sulfonic acid and naphthalene sulfonic acid;, and one or two or more of these can be used.

From the viewpoint of stably forming the polyion complex to produce a highly stable composition and from the viewpoint of using the composition to be produced as a cosmetic composition, the organic acid is preferably one or more selected from the group consisting of aliphatic dicarboxylic acid, hydroxycarboxylic acid, and aromatic sulfonic acid, more preferably one or more selected from the group consisting of succinic acid, lactic acid, malic acid, glycolic acid, p-toluenesulfonic acid, and naphthalenesulfonic acid, even more preferably one or more selected from the group consisting of succinic acid and lactic acid, and even more preferably lactic acid.

At least a part of the organic acid may be in the form of an organic acid salt at the time of blending. The salt of the organic acid is preferably an alkali metal salt or an alkali metal salt of an organic acid, more preferably an alkali metal salt, even more preferably one or more selected from the group consisting of a sodium salt and a potassium salt, and even more preferably a sodium salt, from the viewpoint of use in a cosmetic composition or a fiber treatment composition for head decoration products and from the viewpoint of availability.

The proportion of the organic acid salt in the total amount of the organic acid and the organic acid salt is preferably 50 mass% or less, more preferably 20 mass% or less, even more preferably 5 mass% or less, and may be 0 mass%, from the viewpoint of ease of formation of the polyion complex and improvement in stability of the composition. The proportion (mass%) of the organic acid salt referred to herein means mass% in terms of organic acid.

### (Water)

The water is preferably deionized water or distilled water. Tap water, ground water or the like sterilized with hypochlorous acid or the like may be used as long as the stability of the composition is not impaired.

### (Oily Component)

The emulsified composition obtained by the production method of the present invention contains an oily component as an emulsion-forming component in addition to the cationic polymer (A), the anionic polymer (B), the nonionic polymer (C), the acid (D), and water.

The oily component preferably contains an ionic surfactant and an amphipathic substance having a hydrophobic group from the viewpoint of an texture improving effect of the object to be treated and the improvement of the durability of the effect, in particular, an effect of suppressing entanglement of hair or fibers for head decoration products when the composition is used for treating hair or fibers for head decoration products and the improvement of the durability thereof, and from the viewpoint of the improvement of the moisture resistance. **In** particular, when the emulsified composition is used as a hair cosmetic composition, preferably as a hair rinse, a hair conditioner, a hair treatment, or a hair styling agent, the ionic surfactant is preferably (E) a cationic surfactant, and the amphipathic substance having a hydrophobic group is preferably (F) a higher alcohol. That is, the oily component contained in the emulsified composition preferably contains the cationic surfactant (E) and the higher alcohol (F). Further, the emulsified composition may contain (G) an oil agent and (H) an aqueous medium as optional components.

### [Component (E): Cationic Surfactant]

The cationic surfactant (E) is an oily component contained in the oily phase of the emulsified composition.

Examples of the cationic surfactant include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof.

Examples of (i) an alkyltrimethylammonium salt include an alkyltrimethylammonium salt having an alkyl group having preferably 12 or more and 24 or less carbons and more preferably 16 or more and 24 or less carbons, and specific examples thereof include cetyltrimethylammonium chloride (cetrimonium chloride), stearyltrimethylammonium chloride (steartrimonium chloride), and behenyltrimethylammonium chloride (behentrimonium chloride).

Examples of (ii) an alkoxyalkyltrimethylammonium salt include an alkoxyalkyltrimethylammonium salt having an alkoxy group having preferably 12 or more and 22 or less carbons and more preferably 16 or more and 20 or less carbons, and specific examples thereof include stearoxypropyltrimethylammonium chloride, stearoxyethyltrimethylammonium chloride, and stearoxyhydroxypropyltrimethylammonium chloride.

Examples of (iii) a dialkyldimethylammonium salt include a dialkyldimethylammonium salt having an alkyl group having preferably 12 or more and 22 or less carbons and more preferably 16 or more and 20 or less carbons, and specific examples thereof include distearyldimethylammonium chloride.

Examples of (iv) an alkylamidoalkyltrimethylammonium salt include an alkylamidoalkyltrimethylammonium salt having an alkyl group having preferably 11 or more and 21 or less carbons and more preferably 13 or more and 19 or less carbons, and specific examples thereof include palmitamidopropyltrimethylammonium chloride (palmitamidopropyltrimonium chloride).

(v) Alkyldimethylamine, (vi) alkoxyalkyldimethylamine, and (vii) alkylamidoalkyldimethylamine react with an acid to form a tertiary amine salt, and serve as a cationic surfactant.

The alkyl group in (v) alkyldimethylamine and a salt thereof and the alkoxy group in (vi) alkoxyalkyldimethylamine and a salt thereof are preferably alkyl groups having 12 or more and 22 or less carbons, and more preferably 16 or more and 20 or less carbons.

The alkyl group of the alkylamide moiety in (vii) alkylamidoalkyldimethylamine and a salt thereof is preferably an alkyl group having 11 or more and 21 or less carbons, more preferably 15 or more and 19 or less carbons.

The amines of (v) to (vii) may be caused to react with an acid in advance and blended to the emulsified composition as a salt, or may be blended to the emulsified composition as it is as an amine, then an acid is further blended to the emulsified composition to form a salt in the composition. Thus, herein, the amine and the salt thereof are defined as a cationic surfactant. The content or blending amount thereof is converted in terms of the mass of the amine.

Examples of the salts of the amines of (v) to (vii) include a salt with an organic acid or an inorganic acid. Examples of the organic acid include monocarboxylic acids such as acetic acid and propionic acid; dicarboxylic acids such as malonic acid, succinic acid, glutaric acid, adipic acid, maleic acid, fumaric acid, and phthalic acid; polycarboxylic acids such as polyglutamic acid; hydroxycarboxylic acids such as glycolic acid, lactic acid, hydroxyacrylic acid, glyceric acid, malic acid, tartaric acid, and citric acid; and acidic amino acids such as glutamic acid and aspartic acid. Examples of the inorganic acid include hydrochloric acid, sulfuric acid, and phosphoric acid. Among these, organic acids are preferable, and one or more selected from the group consisting of dicarboxylic acids, hydroxycarboxylic acids, and acidic amino acids are more preferable. The dicarboxylic acid is more preferably at least one selected from the group consisting of maleic acid and succinic acid. The hydroxycarboxylic acid is more preferably one or more selected from the group consisting of glycolic acid, lactic acid, and malic acid. Glutamic acid is more preferable as the acidic amino acid.

Examples of (v) alkyldimethylamine and a salt thereof include N,N-dimethylbehenylamine, N,N-dimethylstearylamine, and organic acid salts thereof, and a lactate salt of N,N-dimethylbehenylamine, a glycolic acid salt of N,N-dimethylstearylamine, and the like are preferable.

Examples of (vi) alkoxyalkyldimethylamine and a salt thereof include N,N-dimethyl-3-hexadecyloxypropylamine, N,N-dimethyl-3-octadecyloxypropylamine, and an organic acid salt thereof, and N,N-dimethyl-3-hexadecyloxypropylamine or a lactate salt thereof, and N,N-dimethyl-3-octadecyloxypropylamine(stearoxypropyldimethylamine) or a lactate salt thereof are preferable.

Examples of (vii) alkylamidoalkyldimethylamine and a salt thereof include N-[3-(dimethylamino)propyl]docosanamide, N-[3-(dimethylamino)propyl]stearamide, and organic acid salts thereof, and lactate salt of N-[3-(dimethylamino)propyl]docosanamide and glycolic acid salt of N-[3-(dimethylamino)propyl]stearamide are preferable.

One or two or more of the component (E) can be used.

Among these, from the viewpoint of further improving the texture of the object to be treated, the component (E) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof, more preferably one or more selected from the group consisting of (i) an alkyltrimethylammonium salt and (vi) alkoxyalkyldimethylamine and a salt thereof, and even more preferably one or more selected from the group consisting of cetyltrimethylammonium chloride, stearyltrimethylammonium chloride, behenyltrimethylammonium chloride, N,N-dimethyl-3-hexadecyloxypropylamine or a lactate salt thereof, and N,N-dimethyl-3-octadecyloxypropylamine-(stearoxypropyldimethylamine) or a lactate salt thereof, even more preferably one or more selected from the group consisting of behenyltrimethylammonium chloride, and N,N-dimethyl-3-octadecyloxypropylamine(stearoxypropyldimethylamine) or a lactate salt thereof.

### [Component (F): Higher Alcohol]

The higher alcohol (F) is an oily component contained in the oily phase of the emulsified composition.

Examples of the component (F) include aliphatic monohydric alcohols having 12 or more carbon atoms, preferably 12 or more and 22 or less carbon atoms. Examples of the higher alcohol include one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol.

Among the above, the component (F) is preferably one or more selected from the group consisting of cetyl alcohol, myristyl alcohol, stearyl alcohol, and behenyl alcohol from the viewpoint of further improving the texture of the object to be treated.

### [Component (G): Oil Agent]

The oil agent (G) is an oily component contained in the oily phase of the emulsified composition other than the components (E) and (F).

Examples of the component (G) include (a) a silicone oil, (b) an ester oil, (c) an ether oil, (d) a hydrocarbon oil, (e) a higher fatty acid, and (f) a wax, and one or two or more of these can be used. Among the above, from the viewpoint of further improving the texture of the object to be treated, one or more selected from the group consisting of (a) a silicone oil, (b) an ester oil, (d) a hydrocarbon oil, and (e) a higher fatty acid, and (a) a silicone oil is more preferable.

The silicone oil (a) is preferably one or more selected from the group consisting of dimethylpolysiloxane (dimethicone), dimethiconol (dimethylpolysiloxane having a hydroxy group at a terminal), methylphenylpolysiloxane, and modified silicone.

Examples of the modified silicone include amino-modified silicone (dimethylpolysiloxane having an amino group, such as amodimethicone), polyether-modified silicone, aminopolyether-modified silicone (silicone having an amino group and a polyether structure), glycerin-modified silicone, carboxy-modified silicone, fatty acid-modified silicone, alcohol-modified silicone, aliphatic alcohol-modified silicone, epoxy-modified silicone, fluorine-modified silicone, and alkyl-modified silicone.

Among the above, from the viewpoint of further improving the texture of the object to be treated, the silicone oil (a) is preferably one or more selected from the group consisting of dimethylpolysiloxane, dimethiconol, methylphenylpolysiloxane, and modified silicone, more preferably one or more selected from the group consisting of dimethylpolysiloxane, dimethiconol, amino-modified silicone, polyether-modified silicone, and aminopolyether-modified silicone, and even more preferably one or more selected from the group consisting of dimethylpolysiloxane, amino-modified silicone, and aminopolyether-modified silicone.

### (Component (H): Aqueous Medium)

The aqueous medium (H) means an aqueous medium other than water. The component (H) is an aqueous component and can be used in both the aqueous composition and the emulsified composition. In the emulsified composition, the component (H) is usually contained in the aqueous phase, but may be blended in the oily phase of the emulsified composition.

Examples of the component (H) include lower alcohols such as ethanol and isopropyl alcohol; low molecular weight diols and triols having 6 or less carbon atoms such as 1,3-butylene glycol, glycerol, ethylene glycol, propylene glycol, and dipropylene glycol. These may be used alone or in combination of two or more.

### (Content of Each component in Composition)

The content of each component in the aqueous composition and the emulsified composition produced by the production method of the present invention is preferably as follows from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of the improvement of the stability of the composition.

The content of the component (A) in the aqueous composition and the emulsified composition is preferably 0.01 mass% or more, more preferably 0.02 mass% or more, even more preferably 0.05 mass% or more, and even more preferably 0.1 mass% or more, from the viewpoint of ease of formation of a polyion complex, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of moisture resistance. From the viewpoint of improving the stability of the composition, the content is preferably 3.0 mass% or less, more preferably 2.5 mass% or less, even more preferably 2.0 mass% or less, even more preferably 1.5 mass% or less, and even more preferably 1.0 mass% or less. The content of the component (A) in the aqueous composition and the emulsified composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, and even more preferably 0.1 mass% or more and 1.0 mass% or less.

The content of the component (B) in the aqueous composition and the emulsified composition is preferably 0.005 mass% or more, more preferably 0.01 mass% or more, and even more preferably 0.02 mass% or more, from the viewpoint of ease of formation of the polyion complex, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of moisture resistance. From the viewpoint of improving the stability of the composition, the content is preferably 3.0 mass% or less, more preferably 2.0 mass% or less, even more preferably 1.5 mass% or less, even more preferably 1.0 mass% or less, even more preferably 0.5 mass% or less, even more preferably 0.3 mass% or less, even more preferably 0.2 mass% or less, and even more preferably 0.1 mass% or less. The content of the component (B) in the aqueous composition and the emulsified composition is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.02 mass% or more and 0.1 mass% or less.

The total content of the component (A) and the component (B) in the aqueous composition and the emulsified composition is preferably 0.02 mass% or more, more preferably 0.04 mass% or more, even more preferably 0.05 mass% or more, and even more preferably 0.1 mass% or more, from the viewpoint of ease of formation of the polyion complex, from the viewpoint of the texture improving effect of the object to be treated and the durability of the effect, in particular, the effect of suppressing entanglement of hair or fibers for head decoration products when used for treating hair or fibers for head decoration products and the improvement of the durability thereof, and from the viewpoint of the improvement of moisture resistance. From the viewpoint of improving the stability of the composition, the content is preferably 5.0 mass% or less, more preferably 4.0 mass% or less, even more preferably 3.0 mass% or less, and even more preferably 2.0 mass% or less. The total content of the component (A) and the component (B) in the aqueous composition and the emulsified composition is preferably 0.02 mass% or more and 5.0 mass% or less, more preferably 0.04 mass% or more and 4.0 mass% or less, even more preferably 0.05 mass% or more and 3.0 mass% or less, and even more preferably 0.1 mass% or more and 2.0 mass% or less.

As the ratio between the contents of the component (A) and the component (B) in the aqueous composition and the emulsified composition, the mass ratio [(A)/(B)] of the component (A) to the component (B) is preferably 1.0 or more, more preferably 2.0 or more, even more preferably 3.0 or more, even more preferably 4.5 or more, even more preferably 4.7 or more, even more preferably 4.8 or more, even more preferably 5.0 or more, even more preferably 5.5 or more, even more preferably 6.0 or more, even more preferably 6.2 or more, and even more preferably 6.5 or more, and preferably 30 or less, more preferably 20 or less, even more preferably 18 or less, even more preferably 16 or less, even more preferably 15 or less, even more preferably 12 or less, and even more preferably 10 or less, from the viewpoint of ease of formation of the polyion complex, from the viewpoint of the texture improving effect of the object to be treated and the improvement of the durability of the effect, in particular, the effect of suppressing entanglement of hair or fibers for head decoration products when the composition is used for treating hair or fibers for head decoration products and the improvement of the durability thereof, and from the viewpoint of the improvement of the moisture resistance. The mass ratio [(A)/(B)] is preferably 1.0 or more and 30 or less, more preferably 1.0 or more and 20 or less, even more preferably 2.0 or more and 20 or less, even more preferably 3.0 or more and 20 or less, even more preferably 4.5 or more and 20 or less, even more preferably 4.7 or more and 18 or less, even more preferably 4.8 or more and 18 or less, even more preferably 5.0 or more and 18 or less, even more preferably 5.5 or more and 16 or less, even more preferably 6.0 or more and 16 or less, even more preferably 6.2 or more and 15 or less, even more preferably 6.5 or more and 15 or less, even more preferably 6.5 or more and 12 or less, and even more preferably 6.5 or more and 10 or less.

The content of the component (C) in the aqueous composition and the emulsified composition is preferably 0.02 mass% or more, more preferably 0.04 mass% or more, even more preferably 0.05 mass% or more, and even more preferably 0.1 mass% or more, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. From the viewpoint of improving the stability of the composition, the content is preferably 5.0 mass% or less, more preferably 4.0 mass% or less, even more preferably 3.0 mass% or less, and even more preferably 2.0 mass% or less. The content of the component (C) in the aqueous composition and the emulsified composition is preferably 0.02 mass% or more and 5.0 mass% or less, more preferably 0.04 mass% or more and 4.0 mass% or less, even more preferably 0.05 mass% or more and 3.0 mass% or less, and even more preferably 0.1 mass% or more and 2.0 mass% or less.

The mass ratio of the component (C) to the total amount of the component (A) and the component (B) [(C)/{(A) + (B)}] in the aqueous composition and the emulsified composition is preferably 0.2 or more, more preferably 0.3 or more, even more preferably 0.4 or more, even more preferably 0.5 or more, even more preferably 0.6 or more, even more preferably 0.8 or more, and preferably 20 or less, more preferably 15 or less, even more preferably 10 or less, even more preferably 8.0 or less, even more preferably 5.0 or less, even more preferably 2.0 or less, even more preferably 1.5 or less from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. The mass ratio [(C)/{(A) + (B)}] is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.

The content of the component (D) in the aqueous composition and the emulsified composition is preferably 0.01 mass% or more, more preferably 0.02 mass% or more from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of the improvement of the stability of the composition. **In** addition, the content is preferably 3.0 mass% or less from the viewpoint of improving the stability of the composition.

When the emulsified composition contains any of (v) alkyldimethylamine and a salt thereof, (vi) alkoxyalkyldimethylamine and a salt thereof, and (vii) alkylamidoalkyldimethylamine and a salt thereof as the cationic surfactant (E), the content of the component (D) in the emulsified composition may be more than 3.0 mass%. This is because the presence of the component (D) as a counter anion of the amine is expected to improve the stability of the emulsified composition and improve the texture of the object to be treated.

When the component (D) is blended in the form of an organic acid salt, the content of the component (D) is an amount converted to an organic acid.

The content of water in the aqueous composition and the emulsified composition is preferably 50 mass% or more, more preferably 70 mass% or more, even more preferably 80 mass% or more, and even more preferably 85 mass% or more from the viewpoint of improving the stability of the composition. The content of water in the aqueous composition may be the balance of the components (A) to (D).

The content of the cationic surfactant (E) in the emulsified composition is preferably 0.1 mass% or more, more preferably 0.2 mass% or more, even more preferably 0.5 mass% or more, and even more preferably 1.0 mass% or more from the viewpoint of further improving the texture of the object to be treated. From the viewpoint of improving the stability of the composition, the content is preferably 10 mass% or less, more preferably 5.0 mass% or less, and even more preferably 3.5 mass% or less. The content of the component (E) in the emulsified composition is preferably 0.1 mass% or more and 10 mass% or less, more preferably 0.2 mass% or more and 5.0 mass% or less, even more preferably 0.5 mass% or more and 5.0 mass% or less, and even more preferably 1.0 mass% or more and 3.5 mass% or less.

The content of the higher alcohol (F) in the emulsified composition is preferably 0.1 mass% or more, more preferably 0.2 mass% or more, even more preferably 0.5 mass% or more, and even more preferably 1.0 mass% or more from the viewpoint of further improving the texture of the object to be treated. From the viewpoint of improving the stability of the composition, the content is preferably 15 mass% or less, more preferably 12 mass% or less, and even more preferably 10 mass% or less. The content of the component (F) in the emulsified composition is preferably 0.1 mass% or more and 15 mass% or less, more preferably 0.2 mass% or more and 12 mass% or less, even more preferably 0.5 mass% or more and 12 mass% or less, and even more preferably 1.0 mass% or more and 10 mass% or less.

When the oil agent (G) is used in the emulsified composition, the content of the component (G) in the emulsified composition is preferably 0.1 mass% or more, more preferably 0.2 mass% or more, and even more preferably 0.5 mass% or more from the viewpoint of further improving the texture of the object to be treated. From the viewpoint of improving the stability of the composition, the content is preferably 20 mass% or less, more preferably 10 mass% or less, and even more preferably 5.0 mass% or less. The content of the component (G) in the emulsified composition is preferably 0.1 mass% or more and 20 mass% or less, more preferably 0.2 mass% or more and 10 mass% or less, even more preferably 0.5 mass% or more and 10 mass% or less, and even more preferably 0.5 mass% or more and 5.0 mass% or less.

When the aqueous medium (H) is used in the aqueous composition and the emulsified composition, the content of the component (H) in the aqueous composition and the emulsified composition is preferably 0.1 mass% or more, more preferably 0.2 mass% or more, even more preferably 0.5 mass% or more, and preferably 10 mass% or less, more preferably 5.0 mass% or less. The content of the component (H) in the composition is preferably 0.1 mass% or more and 10 mass% or less, more preferably 0.2 mass% or more and 5.0 mass% or less, and even more preferably 0.5 mass% or more and 5.0 mass% or less.

In addition, an anionic surfactant, a nonionic surfactant, an amphoteric surfactant, an aromatic ring-containing alcohol, an antioxidant, an anti-dandruff agent, a vitamin agent, a disinfectant, an anti-inflammatory agent, an antiseptic agent, a chelating agent, a moisturizing agent, a pearl agent, ceramides, a perfume, a plant extract, an ultraviolet absorber, a pH adjusting agent, and the like can be blended in the aqueous composition and the emulsified composition.

### (pH)

From the viewpoint of safety, the pH of the composition is preferably 3.0 or more, more preferably 3.2 or more as the pH at 25°C of an aqueous solution obtained by diluting the composition with water by 20 times. From the viewpoint of improving the stability of the composition, the pH is preferably 7.5 or less, more preferably 7.0 or less, even more preferably 6.0 or less, and even more preferably 5.0 or less.

The pH can be measured by the method described in Examples using a pH meter.

Hereinafter, each step of the method for producing the composition according to the present invention will be described. The steps 1 to 3 are common to the methods for producing an aqueous composition and an emulsified composition.

### <Step 1: Preparation of Mixture 1>

In the step 1, a mixture 1 containing (B) an anionic polymer, (C) a nonionic polymer, and water is prepared.

In the preparation of the mixture 1, the order of mixing the components (B) and (C) and water is not particularly limited, and examples thereof include (1) a method of simultaneously adding the components (B) and (C) to water and dissolving or dispersing the components, (2) a method in which a solution obtained by dissolving or dispersing the component (B) in water and a solution obtained by dissolving or dispersing the component (C) in water are prepared and mixed, (3) a method of mixing a solution obtained by dissolving or dispersing the component (B) in water with the component (C), and (4) a method of mixing the component (B) with a solution obtained by dissolving or dispersing the component (C) in water. Among them, (3) a method of mixing a solution obtained by dissolving or dispersing the component (B) in water with the component (C) is preferable from the viewpoint of ease of production.

In the above (2) or (3), when a solution obtained by dissolving or dispersing the component (B) in water is prepared, the component (B) may be dispersed in advance in an aqueous medium other than water, such as dipropylene glycol, and then dissolved or dispersed in water.

In the above (2) to (4), as a solution obtained by dissolving or dispersing the component (B) or the component (C) in water, a commercially available aqueous solution or aqueous dispersion can also be used. For example, a reaction liquid obtained when the component (B) or the component (C) is produced using water as a reaction solvent may be used as a solution obtained by dissolving or dispersing the component (B) or the component (C) in water.

The preparation of the mixture 1 in the step 1 can be performed using a known stirring device such as a homomixer or a mechanical stirrer.

In the step 1, the temperature during preparation of the mixture 1 is not particularly limited, but the temperature is preferably 40°C or more, more preferably 45°C or more, and even more preferably 50°C or more from the viewpoint of improving dissolution or dispersibility of the component (B) and the component (C), and is preferably 90°C or less, more preferably 80°C or less, even more preferably 70°C or less, and even more preferably 65°C or less from the viewpoint of suppressing thermal degradation of the blended components. The temperature during preparation of the mixture 1 is preferably 40°C or more and 90°C or less, more preferably 45°C or more and 80°C or less, even more preferably 50°C or more and 70°C or less, and even more preferably 50°C or more and 65°C or less.

The mixing time at the time of preparing the mixture 1 can be appropriately selected, but is preferably 1 minute or more, more preferably 3 minutes or more, and from the viewpoint of productivity, the time is preferably 6 hours or less.

The total content of the component (B) and the component (C) in the mixture 1 is preferably 0.03 mass% or more, more preferably 0.1 mass% or more, and even more preferably 0.3 mass% or more, and is preferably 50 mass% or less, more preferably 40 mass% or less, and even more preferably 35 mass% or less from the viewpoint of improving the stability of the composition and from the viewpoint of ease of production.

When the method for producing an aqueous composition or the method for producing an emulsified composition is a method including the step 4-2, the total content of the component (B) and the component (C) in the mixture 1 obtained in the step 1 is more preferably 20 mass% or less, more preferably 10 mass% or less, even more preferably 5.0 mass% or less, even more preferably 2.0 mass% or less, and even more preferably 1.0 mass% or less from the viewpoint of improving the stability of the composition.

When the method for producing an aqueous composition or the method for producing an emulsified composition is a method including the step 4-2, the amount of water used in the step 1 is preferably 20 mass% or more, more preferably 25 mass% or more, and preferably 90 mass% or less, more preferably 85 mass% or less, based on 100 mass% of the total amount of the finally obtained composition, from the viewpoint of improving the stability of the composition.

When the method for producing an emulsified composition is a method including the step 4-1, the total content of the component (B) and the component (C) in the mixture 1 obtained in the step 1 is more preferably 0.5 mass% or more and even more preferably 1.0 mass% or more from the viewpoint of the texture improving effect of the object to be treated and the improving of the durability of the effect, in particular, the effect of suppressing entanglement of hair or fibers for head decoration products when the composition is used for treating hair or fibers for head decoration products and the improvement of the durability thereof, and from the viewpoint of the improvement of the moisture resistance, and is even more preferably 80 mass% or less, even more preferably 50 mass% or less, and even more preferably 30 mass% or less from the viewpoint of improving the stability of the composition.

Further, when the method for producing an emulsified composition is a method including the step 4-1, the amount of water used in the step 1 is preferably 1.0 mass% or more, more preferably 3.0 mass% or more, and even more preferably 5.0 mass% or more, and is preferably 50 mass% or less, more preferably 40 mass% or less, and even more preferably 30 mass% or less, based on 100 mass% of the total amount of the finally obtained composition from the viewpoint of improving the stability of the composition.

In the preparation of the mixture 1 in the step 1, optional components other than the cationic polymer (A) and the acid (D) can be appropriately blended, but from the viewpoint of improving the stability of the composition, the total content of the components (B) and (C) and water in the mixture 1 is preferably 70 mass% or more, more preferably 80 mass% or more, even more preferably 90 mass% or more, and even more preferably 95 mass% or more, and 100 mass% or less.

### <Step 2: Preparation of Mixture 2>

In the step 2, the mixture 1 is mixed with (D) an acid or an aqueous solution thereof to prepare a mixture 2 containing the anionic polymer (B), the nonionic polymer (C), the acid (D), and water. Preparation conditions (apparatus used, preparation temperature, mixing time) of the mixture 2 in the step 2 are the same as those in the step 1.

In the production of the aqueous composition, the component (D) used in the step 2 is preferably the total amount of the component (D) used in the production of the composition, and in the production of the emulsified composition, the component (D) used in the step 2 does not need to be the total amount of the component (D) used in the production of the composition but may be at least a part thereof.

In the step 2, water may be added together with the component (D). However, from the viewpoint of improving the stability of the composition, the amount of water used in the step 2 is preferably 0.01 mass% or more, and is preferably 1.0 mass% or less, more preferably 0.5 mass% or less, and even more preferably 0.2 mass% or less, based on 100 mass% of the total amount of the finally obtained composition.

### <Step 3: Preparation of Aqueous Composition (Mixture 3)>

In the step 3, the mixture 2 is mixed with (A) a cationic polymer. Through the step 3, an aqueous composition (mixture 3) containing the cationic polymer (A), the anionic polymer (B), the nonionic polymer (C), the acid (D), and water is obtained.

In the step 3, the cationic polymer (A) may be mixed with the mixture 2 as it is, but from the viewpoint of ease of production, it is preferable to mix a solution obtained by dissolving or dispersing the component (A) in water with the mixture 2.

As the solution obtained by dissolving or dispersing the component (A) in water, a commercially available aqueous solution or aqueous dispersion can also be used. In addition, for example, a reaction liquid when the component (A) is produced using water as a reaction solvent may be used as a solution in which the component (A) is dissolved or dispersed in water.

The other preparation conditions (apparatus used, preparation temperature, mixing time) of the aqueous composition (mixture 3) in the step 3 are the same as in the step 1.

In the step 3, water may be added to adjust the aqueous composition (mixture 3) to a desired concentration.

In the method for producing an aqueous composition, the amount of water used in the step 3 may be an amount such that the total content of the components (A) to (C) in the aqueous composition is preferably in the range described above.

When the method for producing an emulsified composition is a method including the step 4-1, the content of water in the mixture 3 obtained through the step 3 is preferably 5.0 mass% or more, more preferably 10 mass% or more, even more preferably 15 mass% or more, and preferably 50 mass% or less, more preferably 40 mass% or less, based on 100 mass% of the total amount of the finally obtained composition from the viewpoint of improving the stability of the composition.

When the method for producing an emulsified composition is a method including the step 4-2, from the viewpoint of improving the stability of the composition, the content of water in the mixture 3 obtained through the step 3 is preferably 20 mass% or more and 90 mass% or less, based on 100 mass% of the total amount of the finally obtained composition.

### <Step 4: Emulsification Step>

The method for producing an emulsified composition includes, after the step 3, step 4-1 or step 4-2 described below. As a result, it is possible to produce an emulsified composition that has high stability, can improve the texture improving effect of the object to be treated, the durability of the effect, and the moisture resistance, in particular, can improve the effect of suppressing entanglement of hair or fibers for head decoration products and the durability thereof when the emulsified composition is used for treating hair or fibers for head decoration products, and can suppress the spread of hair or fibers for head decoration products after the treatment under high humidity conditions.

Step 4-1: mixing an aqueous phase containing water with an oily phase containing an oily component to prepare an emulsified product, and then mixing the emulsified product with the mixture 3.

Step 4-2: mixing the mixture 3 with an aqueous phase containing water, and then mixing the mixture with an oily phase containing an oily component for emulsification.

### (Step 4-1)

In the step 4-1, an aqueous phase containing water and an oily phase containing an oily component are mixed to prepare an emulsified product, and then the emulsified product is mixed with the mixture 3.

The term "aqueous phase containing water" as used herein is preferably water as the remaining amount. The "oily phase containing an oily component" preferably contains (E) a cationic surfactant and (F) a higher alcohol as oily components, and the oily phase may further contain (H) an aqueous medium, an aromatic ring-containing alcohol, or the like. The total content of the component (E) and the component (F) in the oily phase is preferably 30 mass% or more, more preferably 50 mass% or more, still more preferably 70 mass% or more, and 100 mass% or less, from the viewpoint of stably forming an emulsified product.

The temperature at the time of mixing the aqueous phase and the oily phase in the step 4-1 is not particularly limited, but is preferably 40°C or more, more preferably 45°C or more, and even more preferably 50°C or more from the viewpoint of producing an emulsified composition that is high in stability, can improve the texture improving effect of the object to be treated, the durability of the effect, and the moisture resistance, in particular, can improve the effect of suppressing entanglement of hair or fibers for head decoration products and the durability thereof and can suppress the spread of hair or fibers for head decoration products after the treatment under high humidity conditions when the composition is used for treating hair or fibers for head decoration products, and is preferably 90°C or less, and more preferably 80°C or less from the viewpoint of suppressing thermal degradation of the blending component. The temperature at the time of mixing the aqueous phase and the oily phase in the step 4-1 is preferably 40°C or more and 90°C or less, more preferably 45°C or more and 90°C or less, and even more preferably 50°C or more and 80°C or less. The mixing time of the aqueous phase and the oily phase in the step 4-1 is not particularly limited either, but is preferably 1 minute or more, more preferably 3 minutes or more, from the viewpoint of preparing an emulsified product, and is preferably 1 hour or less from the viewpoint of productivity.

Next, the obtained emulsified product is mixed with the mixture 3.

The emulsified product and the mixture 3 may be mixed at once, or one may be added to the other in a plurality of divided portions and mixed. At the same time as or before or after the mixing of the emulsified product with the mixture 3, the remaining amount of aqueous components other than the mixture 3 (the acid (D), water, the aqueous medium (H), etc.) and oily components other than the components (E) and (F) (the oil agent (G), etc.) may be added and mixed.

The temperature at the time of mixing the emulsified product and the mixture 3 is not particularly limited, but is preferably 50°C or less, more preferably 45°C or less, and preferably 10°C or more, more preferably 20°C or more, from the viewpoint of stability of the resulting emulsified composition. The temperature at the time of mixing the emulsified product and the mixture 3 is preferably 10°C or more and 50°C or less, more preferably 20°C or more and 45°C or less.

The mixing time of the emulsified product and the mixture 3 is not particularly limited either, and is preferably 1 minute or more, more preferably 3 minutes or more, and is preferably 1 hour or less from the viewpoint of productivity.

### (Step 4-2)

In the step 4-2, the mixture 3 is mixed with an aqueous phase containing water, and then mixed with an oily phase containing an oily component to be emulsified. The "aqueous phase containing water" and the "oily phase containing an oily component" referred to herein are the same as in the step 4-1.

The temperature at the time of mixing the aqueous phase containing the mixture 3 and water with the oily phase in the step 4-2 is not particularly limited, but is preferably 40°C or more, more preferably 45°C or more, and even more preferably 50°C or more from the viewpoint of producing an emulsified composition that is high in stability, can improve the texture improving effect of the object to be treated, the durability of the effect, and the moisture resistance, in particular, can improve the effect of suppressing entanglement of hair or fibers for head decoration products and the durability thereof when the composition is used for treating hair or fibers for head decoration products, and can suppress the spread of hair or fibers for head decoration products after the treatment under high humidity conditions, and is preferably 90°C or less, and more preferably 80°C or less from the viewpoint of suppressing thermal degradation of the blending component. The temperature at the time of mixing the aqueous phase containing the mixture 3 and water with the oily phase in the step 4-2 is preferably 40°C or more and 90°C or less, more preferably 45°C or more and 90°C or less, and even more preferably 50°C or more and 80°C or less. The mixing time for the aqueous phase containing the mixture 3 and water and the oily phase in the step 4-2 is not particularly limited either, but is preferably 1 minute or more, more preferably 3 minutes or more, from the viewpoint of preparing an emulsified product, and is preferably 1 hour or less from the viewpoint of productivity.

Next, as necessary, the obtained emulsified product can be mixed with the remaining amount of the aqueous component (the acid (D), water, the aqueous medium (H), and the like) and an oily component (the oil agent (G) or the like) other than the components (E) and (F).

Each of the mixing steps in the step 4-1 and the step 4-2 can be performed using the same apparatus as the apparatus used in the steps 1 to 3.

The method for producing an emulsified composition includes the steps 1 to 3 and the step 4-1 or 4-2. From the viewpoint of simplicity of production, the method for producing an emulsified composition preferably includes the steps 1 to 3 and the step 4-2.

With respect to the above-described embodiments, the present invention further discloses the following aspects.
<1> A method for producing an aqueous composition,
   the aqueous composition comprising (A) a cationic polymer, (B) an anionic polymer, (C) a nonionic polymer, (D) an acid, and water,
   the method somprising steps 1 to 3 described below in this order:
      step 1: preparing a mixture 1 comprising the anionic polymer (B), the nonionic polymer, and water;
      step 2: mixing the mixture 1 with the acid (D) or an aqueous solution thereof to prepare a mixture 2 comprising the anionic polymer (B), the nonionic polymer (C), the acid (D), and water; and
      step 3: mixing the mixture 2 with the cationic polymer (A).
<2> A method for producing an emulsified composition,
   the emulsified composition comprising (A) a cationic polymer, (B) an anionic polymer, (C) a nonionic polymer, (D) an acid, an oily component, and water,
   the method including steps 1 to 3 and step 4-1 or 4-2 described below in this order:
      step 1: preparing a mixture 1 comprising the anionic polymer (B), the nonionic polymer (C), and water;
      step 2: mixing the mixture 1 with the acid (D) or an aqueous solution thereof to prepare a mixture 2 comprising the anionic polymer (B), the nonionic polymer (C), the acid (D), and water;
      step 3: mixing the mixture 2 with the cationic polymer (A) to prepare a mixture 3 comprising the cationic polymer (A), the anionic polymer (B), the nonionic polymer (C), the acid (D), and water;
      step 4-1: mixing an aqueous phase containing water with an oily phase containing an oily component to prepare an emulsified product, and then mixing the emulsified product with the mixture 3; and
      step 4-2: mixing the mixture 3 with an aqueous phase containing water, and then mixing the mixture with an oily phase containing an oily component for emulsification.
<3> The production method according to <2>, wherein the emulsified composition is preferably an oil-in-water emulsified composition.
<4> The production method according to any one of <1> to <3>, wherein the viscosity of a 1 mass% aqueous solution of the component (A) at 30°C is preferably 1000 mPa·s or more, more preferably 1000 mPa·s or more and 100000 mPa·s or less, even more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.
<5> The production method according to any one of <1> to <4>, wherein the component (A) has a cationic charge density of preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.
<6> The production method according to any one of <1> to <5>, wherein the component (A) is one or more selected from the group consisting of a cationized polyvinyl alcohol, a polyethyleneimine, a methacryloylethyltrimethylammonium salt polymer, a quaternized dialkylaminoalkyl (meth)acrylate polymer, a diallyl quaternized ammonium salt polymer, a methacrylamidopropyltrimethylammonium salt polymer, a vinylimidazolium trichloride-vinylpyrrolidone copolymer (polyquaternium-16), a vinylpyrrolidone-alkylamino (meth)acrylate copolymer, a vinylpyrrolidone-alkylamino (meth)acrylate-vinylcaprolactam copolymer, an alkylacrylamide-(meth)acrylate-alkylaminoalkylacrylamide-polyethylene glycol (meth)acrylate copolymer, and an adipic acid-dimethylaminohydroxypropylethylenetriamine copolymer.
<7> The production method according to any one of <1> to <6>, wherein the component (A) is a polymer including a constituent unit represented by General Formula (1) described below: 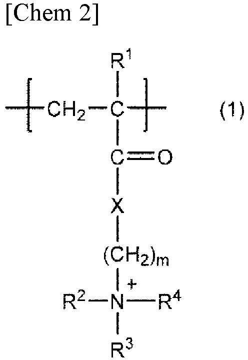 wherein R¹ is a hydrogen atom or a methyl group, and R² to R⁴ are each independently an alkyl group having 1 or more and 4 or less carbons, X is -O- or -NH-, and m is the number of 1 to 4.
<8> The production method according to <7>, wherein a content of the constituent unit represented by General Formula (1) in the component (A) is preferably 8.0 mol% or more, more preferably 10 mol% or more, and is 100 mol% or less, based on all constituent units constituting the component (A).
<9> The production method according to any one of <1> to <8>, wherein the component (A) is preferably one or more selected from the group consisting of a methacryloylethyltrimethylammonium salt polymer and a quaternized dialkylaminoalkyl (meth)acrylate salt polymer, more preferably one or more selected from the group consisting of a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), a methacryloylethyldimethylbetaine-methacryloylethyltrimethylammonium chloride-2-hydroxyethyl methacrylate copolymer (polyquaternium-48), and an N,N-dimethylaminoethylmethacrylic acid diethyl sulfate-N,N-dimethylacrylamide-polyethylene glycol dimethacrylate copolymer (polyquaternium-52), even more preferably one or more selected from the group consisting of a methacryloylethyltrimethylammonium chloride polymer (polyquaternium-37) and an N,N-dimethylaminoethylmethacrylic acid diethyl sulfate-N,N-dimethylacrylamide-polyethylene glycol dimethacrylate copolymer (polyquaternium-52).
<10> The production method according to any one of <1> to <9>, wherein the component (B) contains (B1) one or more selected from the group consisting of a cross-linked polymer containing a constituent unit derived from a (meth)acrylic acid and an anionic polysaccharide.
<11> The production method according to <10>, wherein the crosslinked polymer containing a constituent unit derived from a (meth)acrylic acid is a homopolymer or copolymer of a (meth)acrylic acid having a crosslinked structure, or a salt thereof.
<12> The production method according to <11>, wherein the homopolymer of a (meth)acrylic acid is one or more selected from the group consisting of a polyacrylic acid, a polymethacrylic acid, and a salt thereof.
<13> The production method according to <11>, wherein the copolymer of a (meth)acrylic acid is one or more selected from the group consisting of a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, preferably a (meth)acrylic acid/(meth)acrylic acid alkyl ester, more preferably an acrylic acid/acrylic acid alkyl ester.
<14> The production method according to any one of <10> to <13>, wherein the cross-linked polymer containing a constituent unit derived from a (meth)acrylic acid is one or more selected from the group consisting of a carboxyvinyl polymer or a salt thereof, an (acrylates/alkyl acrylate (C10-30)) crosspolymer, a (Na acrylate/acryloyldimethyltaurine/dimethylacrylamide)crosspolymer, and an acrylates crosspolymer-4.
<15> The production method according to any one of <10> to <14>, wherein the anionic polysaccharide is one or more selected from the group consisting of a polysaccharide having a carboxy group, a sulfate of a polysaccharide, and a salt thereof, preferably a polysaccharide having a carboxy group or a salt thereof, more preferably alginic acid, carboxymethyl cellulose, or a salt thereof, even more preferably alginic acid or a salt thereof.
<16> The production method according to any one of <10> to <15>, wherein the component (B1) is preferably one or more selected from the group consisting of a carboxyvinyl polymer, an (acrylates/alkyl acrylate (C10-30)) crosspolymer, and an anionic polysaccharide, more preferably an anionic polysaccharide or a salt thereof, and even more preferably alginic acid or a salt thereof.
<17> The production method according to any one of <10> to <16>, wherein the component (B1) has an anionic charge density of preferably 1.0 mmol/g or more and 25 mmol/g or less, more preferably 2.0 mmol/g or more and 20 mmol/g or less, even more preferably 3.5 mmol/g or more and 15 mmol/g or less.
<18> The production method according to any one of <10> to <17>, wherein the anionic polysaccharide as the component (B1) has a weight average molecular weight (Mw) of preferably 2000 or more and 3500000 or less, more preferably 10000 or more and 3000000 or less, even more preferably more than 30000 and 3000000 or less, and even more preferably 50000 or more and 3000000 or less.
<19> The production method according to any one of <10> to <18>, wherein a content of the component (B 1) in the component (B) is preferably 50 mass% or more, more preferably 60 mass% or more, even more preferably 70 mass% or more, even more preferably 80 mass% or more, and is 100 mass% or less.
<20> The production method according to any one of <10> to <19>, wherein the component (B) further contains (B2) an anionic polymer other than the component (B1).
<21> The production method according to <20>, wherein the component (B2) has a molecular weight of preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.
<22> The production method according to <20> or <21>, wherein the component (B2) is preferably a polymer having a carboxy group with a molecular weight of 1000 or more and 50000 or less, more preferably 1000 or more and 30000 or less, and more preferably a non-crosslinked polymer containing a constituent unit derived from a (meth)acrylic acid with a molecular weight of 1000 or more and 30000 or less.
<23> The production method according to <22>, wherein the non-crosslinked polymer containing a constituent unit derived from a (meth)acrylic acid is one or more selected from the group consisting of a poly(meth)acrylic acid, a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl) acrylamide copolymer, and a salt thereof.
<24> The production method according to any one of <20> to <23>, wherein the component (B2) is one or more selected from the group consisting of a poly(meth)acrylic acid, a (meth)acrylic acid/(meth)acrylic acid alkyl ester copolymer, and a salt thereof.
<25> The production method according to any one of <20> to <24>, wherein the component (B2) has an anionic charge density of preferably 1.0 mmol/g or more and 25 mmol/g or less, more preferably 3.0 mmol/g or more and 20 mmol/g or less, even more preferably 5.0 mmol/g or more and 15 mmol/g or less.
<26> The production method according to any one of <20> to <25>, wherein a content of the component (B2) in the component (B) is preferably 1 mass% or more and 50 mass% or less, more preferably 2 mass% or more and 40 mass% or less, even more preferably 3 mass% or more and 30 mass% or less, even more preferably 5 mass% or more and 20 mass% or less.
<27> The production method according to any one of <20> to <26>, wherein a mass ratio [(B2)/{(B1) + (B2)}] of the component (B2) to a total amount of the component (B1) and the component (B2) is preferably 0.01 or more and 1.0 or less, more preferably 0.02 or more and 0.80 or less, even more preferably 0.05 or more and 0.50 or less, and even more preferably 0.08 or more and 0.30 or less.
<28> The production method according to any one of <1> to <27>, wherein the component (C) is one or more selected from the group consisting of polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam.
<29> The production method according to <28>, wherein the polyalkylene glycol is preferably one or more selected from the group consisting of polyethylene glycol, polypropylene glycol, and a polyethylene glycol-polypropylene glycol copolymer, and more preferably one or more selected from the group consisting of polyethylene glycol and a polyethylene glycol-polypropylene glycol copolymer.
<30> The production method according to <28>, wherein the polyalkylene glycol derivative is one or more selected from the group consisting of a polyalkylene glycol monoalkyl ether, a polyalkylene glycol dialkyl ether, a polyalkylene glycol monoaryl ether, a polyalkylene glycol diaryl ether, a polyalkylene glycol monofatty acid ester, a polyalkylene glycol difatty acid ester, and a polyalkylene glycol diglycidyl ether, preferably one or more selected from the group consisting of a polyalkylene glycol monoalkyl ether, a polyalkylene glycol dialkyl ether, a polyalkylene glycol monofatty acid ester, and a polyalkylene glycol difatty acid ester, more preferably one or more selected from the group consisting of a polyalkylene glycol monofatty acid ester and a polyalkylene glycol difatty acid ester.
<31> The production method according to any one of <1> to <30>, wherein the component (C) is preferably one or more selected from the group consisting of polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or di-fatty acid ester thereof, and polyvinylpyrrolidone, and even more preferably one or more selected from the group consisting of polyethylene glycol, a polyethylene glycol-polypropylene glycol copolymer, a mono- or di-fatty acid ester thereof, and polyvinylpyrrolidone.
<32> The production method according to any one of <1> to <31>, wherein the component (C) has a weight average molecular weight of preferably 2000 or more and 2000000 or less, more preferably 5000 or more and 1500000 or less, and even more preferably 8000 or more and 150000 or less.
<33> The production method according to any one of <1> to <32>, wherein the component (D) is preferably an organic acid, more preferably one or more selected from the group consisting of a carboxylic acid compound and a sulfonic acid compound other than the component (B).
<34> The production method according to <33>, wherein the organic acid is preferably one or more selected from the group consisting of an aliphatic dicarboxylic acid, a hydroxycarboxylic acid, and an aromatic sulfonic acid, more preferably one or more selected from the group consisting of succinic acid, lactic acid, malic acid, glycolic acid, p-toluenesulfonic acid, and naphthalenesulfonic acid, even more preferably one or more selected from the group consisting of succinic acid and lactic acid, and even more preferably lactic acid.
<35> The production method according to any one of <2> to <34>, wherein the emulsified composition contains an oily component, preferably an oily component containing (E) a cationic surfactant and (F) a higher alcohol.
<36> The production method according to <35>, wherein the component (E) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof, more preferably one or more selected from the group consisting of (i) an alkyltrimethylammonium salt and (vi) alkoxyalkyldimethylamine and a salt thereof, even more preferably one or more selected from the group consisting of cetyltrimethylammonium chloride, stearyltrimethylammonium chloride, behenyltrimethylammonium chloride, N,N-dimethyl-3-hexadecyloxypropylamine or a lactate salt thereof, and N,N-dimethyl-3-octadecyloxypropylamine (stearoxypropyldimethylamine) or a lactate salt thereof, and even more preferably one or more selected from the group consisting of behenyltrimethylammonium chloride and N,N-dimethyl-3-octadecyloxypropylamine (stearoxypropyldimethylamine) or a lactate salt thereof.
<37> The production method according to <35> or <36>, wherein the component (F) is one or more selected from the group consisting of lauryl alcohol, cetyl alcohol, myristyl alcohol, oleyl alcohol, stearyl alcohol, isostearyl alcohol, 2-octyldodecanol, behenyl alcohol and cetostearyl alcohol, preferably one or more selected from the group consisting of cetyl alcohol, myristyl alcohol, stearyl alcohol and behenyl alcohol.
<38> The production method according to any one of <2> to <37>, wherein the emulsified composition further contains (G) an oil agent.
<39> The production method according to <38>, wherein the component (G) is one or more selected from the group consisting of (a) a silicone oil, (b) an ester oil, (c) an ether oil, (d) a hydrocarbon oil, (e) a higher fatty acid, and (f) a wax, preferably one or more selected from the group consisting of (a) a silicone oil, (b) an ester oil, (d) a hydrocarbon oil, and (e) a higher fatty acid, and more preferably (a) a silicone oil.
<40> The production method according to any one of <2> to <39>, wherein the emulsified composition further contains (H) an aqueous medium.
<41> The production method according to <40>, wherein the component (H) is one or more selected from the group consisting of ethanol, isopropyl alcohol, 1,3-butylene glycol, glycerol, ethylene glycol, propylene glycol, and dipropylene glycol.
<42> The production method according to any one of <1> to <41>, wherein a content of the component (A) in the aqueous composition the emulsified composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, and even more preferably 0.1 mass% or more and 1.0 mass% or less.
<43> The production method according to any one of <1> to <42>, wherein a content of the component (B) in the aqueous composition and the emulsified composition is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.02 mass% or more and 0.1 mass% or less.
<44> The production method according to any one of <1> to <43>, wherein a total content of the component (A) and the component (B) in the aqueous composition and the emulsified composition is preferably 0.02 mass% or more and 5.0 mass% or less, more preferably 0.04 mass% or more and 4.0 mass% or less, even more preferably 0.05 mass% or more and 3.0 mass% or less, and even more preferably 0.1 mass% or more and 2.0 mass% or less.
<45> The production method according to any one of <1> to <44>, wherein a mass ratio [(A)/(B)] of the component (A) to the component (B) in the aqueous composition and the emulsified composition is preferably 1.0 or more and 30 or less, more preferably 1.0 or more and 20 or less, even more preferably 2.0 or more and 20 or less, even more preferably 3.0 or more and 20 or less, even more preferably 4.5 or more and 20 or less, even more preferably 4.7 or more and 18 or less, even more preferably 4.8 or more and 18 or less, even more preferably 5.0 or more and 18 or less, even more preferably 5.5 or more and 16 or less, even more preferably 6.0 or more and 16 or less, even more preferably 6.2 or more and 15 or less, even more preferably 6.5 or more and 15 or less, even more preferably 6.5 or more and 12 or less, and even more preferably 6.5 or more and 10 or less.
<46> The production method according to any one of <1> to <45>, wherein a content of the component (C) in the aqueous composition or the emulsified composition is preferably 0.02 mass% or more and 5.0 mass% or less, more preferably 0.04 mass% or more and 4.0 mass% or less, even more preferably 0.05 mass% or more and 3.0 mass% or less, and even more preferably 0.1 mass% or more and 2.0 mass% or less.
<47> The production method according to any one of <1> to <46>, wherein a mass ratio [(C)/{(A) + (B)}] of the component (C) to a total amount of the component (A) and the component (B) in the aqueous composition and the emulsified composition is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.
<48> The production method according to any one of <1> to <47>, wherein a content of the component (D) in the aqueous composition and the emulsified composition is preferably 0.01 mass% or more, more preferably 0.02 mass% or more, and preferably 3.0 mass% or less.
<49> The production method according to any one of <1> to <48>, wherein a content of water in the aqueous composition and the emulsified composition is preferably 50 mass% or more, more preferably 70 mass% or more, even more preferably 80 mass% or more, and even more preferably 85 mass% or more.
<50> The production method according to any one of <35> to <49>, wherein a content of the component (E) in the emulsified composition is preferably 0.1 mass% or more and 10 mass% or less, more preferably 0.2 mass% or more and 5.0 mass% or less, even more preferably 0.5 mass% or more and 5.0 mass% or less, and even more preferably 1.0 mass% or more and 3.5 mass% or less.
<51> The production method according to any one of <35> to <50>, wherein a content of the component (F) in the emulsified composition is preferably 0.1 mass% or more and 15 mass% or less, more preferably 0.2 mass% or more and 12 mass% or less, even more preferably 0.5 mass% or more and 12 mass% or less, and even more preferably 1.0 mass% or more and 10 mass% or less.
<52> The production method according to any one of <38> to <51>, wherein a content of the component (G) in the emulsified composition is preferably 0.1 mass% or more and 20 mass% or less, more preferably 0.2 mass% or more and 10 mass% or less, even more preferably 0.5 mass% or more and 10 mass% or less, and even more preferably 0.5 mass% or more and 5.0 mass% or less.
<53> The production method according to any one of <40> to <52>, wherein a content of the component (H) in the aqueous composition and the emulsified composition is preferably 0.1 mass% or more and 10 mass% or less, more preferably 0.2 mass% or more and 5.0 mass% or less, and even more preferably 0.5 mass% or more and 5.0 mass% or less.
<54> The production method according to any one of <1> to <53>, wherein an aqueous solution obtained by diluting the aqueous composition and the emulsified composition by 20 times with water has a pH at 25°C of preferably 3.0 or more, more preferably 3.2 or more, and preferably 7.5 or less, more preferably 7.0 or less, even more preferably 6.0 or less, and even more preferably 5.0 or less.
<55> The production method according to any one of <1> to <54>, wherein a temperature during preparation of the mixture 1 in the step 1 is preferably 40°C or more and 90°C or less, more preferably 45°C or more and 80°C or less, even more preferably 50°C or more and 70°C or less, and even more preferably 50°C or more and 65°C or less.
<56> The production method according to any one of <1> to <55>, wherein a total content of the component (B) and the component (C) in the mixture 1 is preferably 0.03 mass% or more, more preferably 0.1 mass% or more, and even more preferably 0.3 mass% or more, and is preferably 50 mass% or less, more preferably 40 mass% or less, and even more preferably 35 mass% or less.
<57> The production method according to <56>, wherein when the production method is a method including the step 4-2, a total content of the component (B) and the component (C) in the mixture 1 obtained in the step 1 is more preferably 20 mass% or less, more preferably 10 mass% or less, even more preferably 5 mass% or less, even more preferably 2.0 mass% or less, and even more preferably 1.0 mass% or less.
<58> The production method according to any one of <1> to <57>, wherein when the production method is a method including the step 4-2, an amount of water used in the step 1 is preferably 20 mass% or more, more preferably 25 mass% or more, and preferably 90 mass% or less, more preferably 85 mass% or less, based on 100 mass% of a total amount of a finally obtained composition.
<59> The production method according to <56>, wherein when the production method is the method for producing the emulsified composition including the step 4-1, a total content of the component (B) and the component (C) in the mixture 1 obtained in the step 1 is more preferably 0.5 mass% or more, more preferably 1.0 mass% or more, and even more preferably 80 mass% or less, even more preferably 50 mass% or less, and even more preferably 30 mass% or less.
<60> The production method according to any one of <2> to <59>, wherein when the production method is the method for producing the emulsified composition including the step 4-1, an amount of water used in the step 1 is preferably 1.0 mass% or more, more preferably 3.0 mass% or more, and even more preferably 5.0 mass% or more, and is preferably 50 mass% or less, more preferably 40 mass% or less, and even more preferably 30 mass% or less, based on 100 mass% of a total amount of a finally obtained composition.
<61> The production method according to any one of <2> to <60>, wherein when the production method is the method for producing the emulsified composition including the step 4-1, a content of water in the mixture 3 obtained through the step 3 is preferably 5.0 mass% or more, more preferably 10 mass% or more, even more preferably 15 mass% or more, and preferably 50 mass% or less, more preferably 40 mass% or less, based on 100 mass% of a total amount of a finally obtained composition.
<62> The production method according to any one of <2> to <61>, wherein the method for producing the emulsified composition includes the steps 1 to 3 and the step 4-2.

### Examples

The present invention will be described below through examples, but the present invention is not limited to the scope of the examples. Each measurement and evaluation in Examples were performed by the following methods.

### (Measurement of Viscosity of 1 mass% Aqueous Solution of Cationic Polymer)

A 1 mass% aqueous solution of the cationic polymer used in each example was prepared, and the aqueous solution viscosity at 30°C was measured using a B-type viscometer (TV-20 manufactured by Toki Sangyo Co., Ltd.).

### (pH Measurement)

The hair conditioner of each example was diluted by 20 times with water, and the pH at 25°C was measured using a pH meter (F-51, manufactured by Horiba, Ltd.).

### (Appearance and Texture of Hair Conditioner)

After 2 days from the blending, 1 g of the hair conditioner of each example was dropped on a plastic black underlay, and the appearance when being traced 10 times with a finger and the texture when being crushed with a finger were evaluated according to the following criteria.
A: Aggregated particles were not visually recognized, and the texture was slippery when crushed with a finger
B: Aggregated particles were not visually recognized, but the texture was rough when crushed with a finger
C: Aggregated particles were visually recognized, and the texture was rough when crushed with a finger
D: Separated

### (Measurement of Combing Force After Hair Conditioner Treatment + Two Times of Hair Washing)

(1) "Kao Foam Hair Bleach L" (first agent) 7 mL and "Kao Foam Hair Color d" (second agent) 15 mL manufactured by Kao Corporation were mixed, and the mixture was applied to a tress of Japanese hair having a length of 20 cm and a mass of 30 g. After being left to stand for 20 minutes, it was sufficiently rinsed with warm water at 35 to 40°C (bleaching treatment). The bleaching treatment was repeated four times to prepare a damaged tress for evaluation. The tress was washed with a plain shampoo having the following composition and moistened with water at 15 g ± 0.5 g.
(2) The hair conditioner of each example was applied to the water-containing tress by 1.5 mL and rubbed in by hand, then the tress was allowed to stand for 30 seconds, followed by rinsing with warm water at 35 to 40°C for 15 seconds. Next, the front and back surfaces of the tress were rubbed and washed with the plain shampoo 1 mL for 30 seconds 20 times for each surface and rinsed for 15 seconds.
(3) A plain conditioner 1 mL having the following composition was applied, rubbed in by hand for 30 seconds, and rinsed for 15 seconds. Next, the front and back surfaces of the tress were rubbed and washed with the plain shampoo 1 mL for 30 seconds 20 times for each surface and rinsed for 15 seconds.
(4) After the tress was combed by a brush and entanglement was completely removed, the water content of the tress after rinsing was set to 15 g ± 0.5 g, then the tress was set in a combing force measuring device ("KOT-0303" manufactured by Utsunomiya Seiki.), two brushes (Kao Lunette) were passed through the top of the tress, combing was performed at a constant speed (60 rpm), and the load (g) applied when the combs were passed through to the hair tip was measured. Combing was performed 50 times, and the average value of the combing loads at 50 points in total was taken as the value of "Combing force immediately after treatment". A smaller value of the combing force indicates a better result. The combing load of the damaged tress (containing 15 g ± 0.5 g of water) that was not subjected to the treatment (2) was 2055 g, and when the combing force was 1900 g or less, the hair washing resistance was determined to be good.

[Plain Shampoo Composition] *The blending amount is an active ingredient amount.

| | (mass%) |
|---|---|
| Sodium polyoxyethylene (2.0) lauryl ether sulfate (*1) | : 15.5 |
| Diethanolamide laurate (*2) | : 2.2 |
| Disodium edetate | : 0.15 |
| Sodium benzoate | : 0.18 |
| Oxybenzone | : 0.03 |
| Phosphoric acid | : 0.07 |
| Sodium chloride | : 0.8 |
| Fragrance | : 0.4 |
| Purified water | : balance |
| Total | : 100.00 |

| | |
|---|---|
| (*1) 42.0 mass% as "EMAL E-27C" (active ingredient amount 27 mass%) manufactured by Kao Corporation (*2) AMINON C-11S (manufactured by Kao Corporation), active ingredient amount 100 mass% | |

[Composition of Plane Conditioner] *Blending amount is an active ingredient amount.

| | (mass%) |
|---|---|
| Stearoxypropyldimethylamine (*3) | 0.81 |
| Stearyl alcohol (*4) | 3.09 |
| Lactic Acid (*5) | 0.21 |
| Purified water: | balance |
| Total | : 100.00 |

| | |
|---|---|
| (*3) "FARMIN DM-E80" manufactured by Kao Corporation, active ingredient amount 90 mass% (*4) "KALCOL 8098" manufactured by Kao Corporation, active ingredient amount 100 mass% (*5) "PURAC ULTRAPURE90" manufactured by PURAC Thailand Ltd., active ingredient amount 90 mass% | |

### (Moisture Resistance of Hair after Treatment)

"Kao Foam Hair Bleach L" (first agent) 7 mL and "Kao Foam Hair Color d" (second agent) 15 mL manufactured by Kao Corporation were mixed, and the mixture was applied to a tress of Japanese hair having a length of 30 cm and a mass of 20 g. After being left to stand for 20 minutes, it was sufficiently rinsed with warm water at 35 to 40°C (bleaching treatment). The bleaching treatment was repeated four times to prepare a damaged tress for evaluation.

After the tresses were washed with the plain shampoo, the hair conditioner 1 mL of each example was applied and rubbed in for 30 seconds, left to stand in this state for 1 minute, rinsed with running water at 35 to 40°C for 30 seconds, and dried with a dryer until completely dried.

Using a flat iron ("AHI-938" manufactured by Miki Denki Sangyo) set at 160°C, the tress after drying was subjected to straight setting 10 times while a comb was passed through, and a photograph of the tress immediately after setting was taken. Next, the hair after straight setting was suspended and left to stand for 30 minutes under a high-humidity environment of 35°C and 80% RH, and a photograph was taken again. In the two photographs, the tress width at the 23.5 cm from the root of the tress was measured, and the change in the tress width between immediately after straight setting and after high-humidity storage was calculated from the following equation. Tress width change value = (tress width after storage under high humidity)/(tress width immediately after straight setting)

When the value was 1.3 or more, it appeared to be spread, and thus it was determined that the moisture resistance was low.

### Examples 1 to 11 (Production of Hair Conditioner (Aqueous Composition))

A hair conditioner (aqueous composition) was prepared by the following procedure using the components in the amounts listed in Table 1.

### <Step 1>

An anionic polymer (component (B1) or components (B 1) and (B2)) dispersed in dipropylene glycol in advance and dipropylene glycol (component (H)) were added to a 500 mL beaker containing water, and heated and stirred at 56°C using a stirring device (mechanical stirrer equipped with two propeller blades). After confirming that the anionic polymer was completely dissolved, the nonionic polymer (C) was further added and completely dissolved to obtain a mixture 1.

### <Step 2>

The acid (D) was added to the mixture 1 and stirred to obtain a mixture 2.

### <Step 3>

The cationic polymer (A) dispersed in water in advance was added to the mixture 2. The mixture was heated and stirred at 56°C to completely dissolve the cationic polymer to obtain a hair conditioner.

The obtained hair conditioner was subjected to various evaluations according to the methods described above. The results are shown in Table 1.

### Comparative Example 1

For the step 1, the same operation as in Examples 1 to 11 was performed to obtain the mixture 1.

The cationic polymer (A) dispersed in water in advance was added to the mixture 1. After the cationic polymer was completely dissolved by heating and stirring at 56°C, the acid (D) was added and stirred to obtain a hair conditioner.

The obtained hair conditioner was subjected to various evaluations according to the methods described above. The results are shown in Table 2.

### Comparative Example 2

The acid (D) was added to a 500 mL beaker containing water. Next, anionic polymers (components (B1) and (B2)) in a state of being dispersed in dipropylene glycol in advance were added, and heating and stirring were performed at 56°C using a stirring device. After confirming that the anionic polymer was completely dissolved, the nonionic polymer (C) was further added and completely dissolved to obtain a mixture.

The cationic polymer (A) dispersed in water in advance was added to the mixture. The mixture was heated and stirred at 56°C to completely dissolve the cationic polymer to obtain a hair conditioner.

The obtained hair conditioner was subjected to various evaluations according to the methods described above. The results are shown in Table 2.

### Comparative Example 3

The acid (D) was added to a 500 mL beaker containing water. Next, the cationic polymer (A) dispersed in water in advance was added. The mixture was heated and stirred at 56°C using a stirring device to completely dissolve the cationic polymer and obtain a mixture.

To the mixture, the anionic polymer (B) (components (B 1) and (B2)) in a state of being dispersed in dipropylene glycol in advance was added, and heating and stirring were performed at 56°C using a stirring device. After confirming that the anionic polymer was completely dissolved, the nonionic polymer (C) was further added and completely dissolved to obtain a hair conditioner.

The obtained hair conditioner was subjected to various evaluations according to the methods described above. The results are shown in Table 2.

### Comparative Example 4

The cationic polymer (A) was added to a 500 mL beaker containing water. The mixture was heated and stirred at 56°C using a stirring device to completely dissolve the cationic polymer.

Next, anionic polymers (components (B1) and (B2)) in a state of being dispersed in dipropylene glycol in advance were added, and heating and stirring were performed at 56°C. After confirming that the anionic polymer was completely dissolved, the nonionic polymer (C) was further added and completely dissolved to obtain a mixture.

The acid (D) was added to the mixture and stirred to obtain a hair conditioner.

The obtained hair conditioner was subjected to various evaluations according to the methods described above. The results are shown in Table 2.

### Comparative Example 5

The cationic polymer (A) was added to a 500 mL beaker containing water. The mixture was heated and stirred at 56°C using a stirring device to completely dissolve the cationic polymer.

To the mixture, the acid (D) was added and stirred, then anionic polymers (components (B1) and (B2)) in a state of being dispersed in dipropylene glycol in advance were added, and heating and stirring were performed at 56°C. After confirming that the anionic polymer was completely dissolved, the nonionic polymer (C) was further added and completely dissolved to obtain a hair conditioner.

The obtained hair conditioner was subjected to various evaluations according to the methods described above. The results are shown in Table 2.

### Example 12 (Production of Hair Conditioner (Emulsified Composition))

A hair conditioner (emulsified composition) was prepared by the following procedure using the components in the amounts listed in Table 3.

### <Step 1>

An anionic polymer (component (B1) or components (B1) and (B2)) dispersed in dipropylene glycol (100 mass% of the total amount) in advance was added to a 500 mL beaker containing water, and heated and stirred at 56°C using a stirring device (mechanical stirrer equipped with two propeller blades). After confirming that the anionic polymer was completely dissolved, the nonionic polymer (C) was further added and completely dissolved to obtain a mixture 1.

### <Step 2>

The acid (D) was added to the mixture 1 and stirred to obtain a mixture 2.

### <Step 3>

The cationic polymer (A) dispersed in water in advance was added to the mixture 2. The mixture was heated and stirred at 56°C to completely dissolve the cationic polymer, thereby obtaining an aqueous composition (mixture 3).

### <Step 4-2>

To the mixture 3, the remaining amount of water heated to 56°C was added. Next, (E) a cationic surfactant, (F) a higher alcohol, the remaining dipropylene glycol and benzyl alcohol were mixed in advance and dissolved at 85°C to obtain an oily phase, which was then added and stirred at 56°C for 10 minutes. After stirring for 10 minutes, heating was terminated, and air cooling was started. The remaining amount of the acid (D) (90% lactic acid) and polydimethylsiloxane were added thereto, and the mixture was cooled while being stirred until the liquid temperature became 40°C or less, whereby a hair conditioner was obtained.

The obtained hair conditioner was subjected to various evaluations according to the methods described above. The results are shown in Table 3.

### Examples 13 to 16

### <Steps 1 to 3>

For the steps 1 to 3, the same operation as in Example 12 was performed to obtain an aqueous composition (mixture 3). The amount of water used in the step 1 was the amount shown in Table 3.

### <Step 4-1>

To the aqueous composition (mixture 3) obtained in the step 3, the remaining amount of water heated to 56°C, the cationic surfactant (E), the higher alcohol (F), the remaining dipropylene glycol, and benzyl alcohol were preliminarily mixed and dissolved at 85°C to obtain an oily phase, which was then added and stirred at 56°C for 10 minutes (preparation of emulsified product). After stirring for 10 minutes, heating was terminated, and air cooling was started. The mixture 3 obtained through the step 3, the remaining amount of the acid (D) (90% lactic acid), and polydimethylsiloxane were added thereto, and the mixture was cooled while being stirred until the liquid temperature became 40°C or less, whereby a hair conditioner was obtained.

The obtained hair conditioner was subjected to various evaluations according to the methods described above. The results are shown in Table 3.

### Table 1

**Table 1**

| (mass%) | | | | | Examples | | | | | | | | | | |
|---|---|---|---|---|---|---|---|---|---|---|---|---|---|---|---|
| | | | | | 1 | 2 | 3 | 4 | 5 | 6 | 7 | 8 | 9 | 10 | 11 |
| | (B) | (B1) | Sodium alginate | KIMICA ALGIN I-3-150 (charge density 4.65 mmol/g) *1 (1% viscosity 300 to 400 mPa·s) | 0.041 | 0.041 | 0.041 | 0.042 | 0.041 | 0.041 | 0.041 | | | 0.041 | 0.041 |
| | | | Carboxyvinyl polymer | Carbopol 980 (charge density 13.88 mmol/g) *2 | | | | | | | | 0.041 | | | |
| | | | (Acrylates/alkyl acrylate (C10-30)) crosspolymer | Pemulen TR-1 (charge density 13.67 mmol/g) *3 | | | | | | | | | 0.041 | | |
| | | (B2) | (Acrylic acid/stearyl acrylate) copolymer | SOFCARE SA-37W *4 (charge density 8.88 mmol/g, Mw 20000) | | 0.006 | 0.006 | | 0.006 | 0.006 | 0.006 | | | | |
| | | | Polyacrylic acid | Jurymer AC-10L (charge density 13.88 mmol/g, Mw 20000 to 30000) *5 | | | | 0.004 | | | | | | | |
| Step 1 | (C) | | Polyethylene glycol | BLAUNON PEG-20000S *6 | 0.40 | 0.40 | 0.40 | 0.40 | 0.40 | 0.40 | | 0.40 | 0.40 | | |
| | | | Polyvinylpyrrolidone | Luviskol K-90 *7 | | | | | | | 0.40 | | | | |
| | | | Polyoxyethylene polyoxypropylene glycol (150E.O.)(35P.O.) | PLONON #208 (PEG/PPG-150/35 copolymer) *8 | | | | | | | | | | 0.40 | |
| | | | Polyoxyethylene polyoxypropylene glycol (240E.O.)(60P.O.) | Unilube 75DE-2620R (PEG-PPG-240/60 copolymer) *9 | | | | | | | | | | | 0.40 |
| | (H) | | Dipropylene glycol | DPG-RF *10 | 2 | 2 | 2 | 2 | 2 | 2 | 2 | 2 | 2 | 2 | 2 |
| | Water | | | Ion exchange water | 82.51 | 82.44 | 82.53 | 82.41 | 88.42 | 79.55 | 82.49 | 82.50 | 82.50 | 82.50 | 82.50 |
| Step 2 | (D) | | Lactic acid | 90% Lactic acid *11 | 0.315 | 0.315 | | 0.315 | 0.315 | 0.315 | 0.315 | 0.315 | 0.315 | 0.315 | 0.315 |
| | | | Hydrochloric acid | 1N-hydrochloric acid | | | 0.04 | | | | | | | | |
| Step 3 | (A) | | Polyquaternium-52 | SOFCARE KG-101W-E (charge density 0.83 mmol/g) *12 (1% viscosity 2560 mPa·s) | 0.353 | 0.353 | 0.353 | 0.355 | | | 0.353 | 0.353 | 0.353 | 0.353 | 0.353 |
| | | | Polyquaternium-52 | SOFCARE KG-301W (charge density 1.84 mmol/g) *13 (1% viscosity 2070 mPa·s) | | | | | 0.353 | | | | | | |
| | | | Polyquaternium-37 | Ultragel 300 (charge density 4.81 mmol/g) *14 (1% viscosity 17650 mPa·s) | | | | | | 0.353 | | | | | |
| | Water | | | Ion exchange water | Balance | Balance | Balance | Balance | Balance | Balance | Balance | Balance | Balance | Balance | Balance |
| Total | | | | | 100 | 100 | 100 | 100 | 100 | 100 | 100 | 100 | 100 | 100 | 100 |
| Order of steps 1 to 3 | | | | | 1 → 2 → 3 | 1 → 2 → 3 | 1 → 2 → 3 | 1 → 2 → 2 | 1 → 2 → 3 | 1 → 2 → 2 | 1 → 2 → 3 | 1 → 2 → 3 | 1 → 2 → 3 | 1 → 2 → 3 | 1 → 2 → 3 |
| Concentration (mass%) of mixture 1 prepared in step 1 | | | | | 2.87 | 2.88 | 2.88 | 2.88 | 2.69 | 2.98 | 2.88 | 2.87 | 2.87 | 2.87 | 2.87 |
| Amount of water used in step 1 (mass% in composition as final product) | | | | | 82.51 | 82.49 | 82.58 | 82.41 | 88.42 | 79.55 | 82.49 | 82.50 | 82.50 | 82.50 | 82.50 |
| Amount of water used in step 2 (mass% in composition as final product) | | | | | 0.04 | 0.04 | 0.07 | 0.04 | 0.04 | 0.04 | 0.04 | 0.04 | 0.04 | 0.04 | 0.04 |
| Amount of water used in step 3 (mass% in composition as final product) | | | | | 13.61 | 13.61 | 13.61 | 13.68 | 2.27 | 17.30 | 13.61 | 13.61 | 13.61 | 13.61 | 13.61 |
| pH (25°C, at 20-fold dilution) | | | | | 3.4 | 3.4 | 3.4 | 3.4 | 3.4 | 3.4 | 3.4 | 3.4 | 3.4 | 3.4 | 3.4 |
| Total amount of component (A) (mass%) | | | | | 0.353 | 0.353 | 0.353 | 0.355 | 0.353 | 0.353 | 0.353 | 0.353 | 0.353 | 0.353 | 0.353 |
| Total amount of component (B) (mass%) | | | | | 0.041 | 0.047 | 0.047 | 0.046 | 0.047 | 0.047 | 0.047 | 0.041 | 0.041 | 0.041 | 0.041 |
| Total amount of component (A) and component (B) (mass%) | | | | | 0.394 | 0.400 | 0.400 | 0.401 | 0.400 | 0.400 | 0.400 | 0.394 | 0.394 | 0.394 | 0.394 |
| Cation (A)/anion (B1)/anion (B2) charge ratio (molar ratio) | | | | | 60/40/0 | 55/36/9 | 55/36/9 | 54/36/10 | 73/21/6 | 87/10/3 | 54.6/35.5/9.9 | 34/66/0 | 34/66/0 | 60/40/0 | 60/40/0 |
| Mass ratio (A)/(B) | | | | | 8.61 | 7.55 | 7.55 | 7.72 | 7.51 | 7.51 | 7.51 | 8.61 | 8.61 | 8.61 | 8.61 |
| Content of component (B1) in component (B) (mass%) | | | | | 100.00 | 88.03 | 88.03 | 91.30 | 87.23 | 87.23 | 87.23 | 100.00 | 100.00 | 100.00 | 100.00 |
| Content of component (B2) in component (B) (mass%) | | | | | 0.00 | 11.97 | 11.97 | 8.70 | 12.77 | 12.77 | 12.77 | 0.00 | 0.00 | 0.00 | 0.00 |
| Mass ratio (B2)/{(B1) + (B2)} | | | | | 0.00 | 0.12 | 0.12 | 0.09 | 0.13 | 0.13 | 0.13 | 0.00 | 0.00 | 0.00 | 0.00 |
| Total amount of component (C) (mass%) | | | | | 0.40 | 0.40 | 0.40 | 0.40 | 0.40 | 0.40 | 0.40 | 0.40 | 0.40 | 0.40 | 0.40 |
| Mass ratio (C)/{(A) + (B)} | | | | | 1.02 | 1.00 | 1.00 | 1.00 | 1.00 | 1.00 | 1.00 | 1.02 | 1.02 | 1.02 | 1.02 |
| Evaluation results | Appearance and texture of hair conditioner after 2 days from blending | | | A: Aggregated particles are not visually recognized, and are slippery when crushed | A | A | A | A | A | A | A | C | C | A | A |
| | | | | B: Aggregated particles are not visually recognized, and are rough when crushed | | | | | | | | | | | |
| | | | | C: Aggregated particles are visually recognized | | | | | | | | | | | |
| | | | | D: Separated | | | | | | | | | | | |
| | Combing force after hair conditioner treatment + two times of hair washing | | | Standard: 1900 g or less *blank = 2055 g | 1449 | 1385 | 1642 | 1383 | 1468 | 1418 | 1611 | 1706 | 1744 | 1527 | 1822 |
| | Moisture resistance of hair after treatment | | | Standard: less than 1.3 | 1.14 | 1.08 | 1.23 | 1.00 | 1.06 | 1.00 | 1.00 | 1.00 | 1.10 | 1.00 | 1.10 |

### Table 2

**Table 2**

| (mass%) | | | | | Comparative Examples | | | | |
|---|---|---|---|---|---|---|---|---|---|
| | | | | | 1 | 2 | 3 | 4 | 5 |
| | (B) | (B1) | Sodium alginate | KIMICA ALGIN I-3-150 (charge density 4.65 mmol/g) *1 (1% viscosity 300 to 400 mPa·s) | 0.041 | 0.041 | 0.041 | 0.041 | 0.041 |
| | | | Carboxyvinyl polymer | Carbopol 980 (charge density 13.88 mmol/g) *2 | | | | | |
| | | | (Acrylates/alkyl acrylate (C10-30)) crosspolymer | Pemulen TR-1 (charge density 13.67 mmol/g) *3 | | | | | |
| | | (B2) | (Acrylic acid/stearyl acrylate) copolymer | SOFCARE SA-37W *4 (charge density 8.88 mmol/g, Mw 20000) | 0.006 | 0.006 | 0.006 | 0.006 | 0.006 |
| | | | Polyacrylic acid | Jurymer AC-10L (charge density 13.88 mmol/g, Mw 20000 to 30000) *5 | | | | | |
| Step 1 | (C) | | Polyethylene glycol | BLAUNON PEG-20000S *6 | 0.40 | 0.40 | 0.40 | 0.40 | 0.40 |
| | | | Polyvinylpyrrolidone | Luviskol K-90 *7 | | | | | |
| | | | Polyoxyethylene polyoxypropylene glycol (150E.O.)(35P.O.) | PLONON #208 (PEG/PPG-150/35 copolymer) *8 | | | | | |
| | | | Polyoxyethylene polyoxypropylene glycol (240E.O.)(60P.O.) | Unilube 75DE-2620R (PEG-PPG-240/60 copolymer) *9 | | | | | |
| | (H) | | Dipropylene glycol | DPG-RF *10 | 2 | 2 | 2 | 2 | 2 |
| | Water | | | Ion exchange water | 82.44 | 82.44 | 82.44 | 82.44 | 82.44 |
| Step 2 | (D) | | Lactic acid | 90% Lactic acid *11 | 0.315 | 0.315 | 0.315 | 0.315 | 0.315 |
| | | | Hydrochloric acid | 1N-hydrochloric acid | | | | | |
| Step 3 | (A) | | Polyquaternium-52 | SOFCARE KG-101W-E (charge density 0.83 mmol/g) *12 (1% viscosity 2560 mPa·s) | 0.353 | 0.353 | 0.353 | 0.353 | 0.353 |
| | | | Polyquaternium-52 | SOFCARE KG-301W (charge density 1.84 mmol/g) *13 (1% viscosity 2070 mPa·s) | | | | | |
| | | | Polyquaternium-37 | Ultragel 300 (charge density 4.81 mmol/g) *14 (1% viscosity 17650 mPa·s) | | | | | |
| | Water | | | Ion exchange water | Balance | Balance | Balance | Balance | Balance |
| Total | | | | | 100 | 100 | 100 | 100 | 100 |
| Order of steps 1 to 3 | | | | | 1 → 3 → 2 | 2 → 1 → 3 | 2 → 3 → 1 | 3 → 1 → 2 | 3 → 2 → 1 |
| Concentration (mass%) of mixture 1 prepared in step 1 | | | | | 2.88 | 2.88 | 2.88 | 2.88 | 2.88 |
| Amount of water used in step 1 (mass% in composition as final product) | | | | | 82.49 | 82.49 | 82.49 | 82.49 | 82.49 |
| Amount of water used in step 2 (mass% in composition as final product) | | | | | 0.04 | 0.04 | 0.04 | 0.04 | 0.04 |
| Amount of water used in step 3 (mass% in composition as final product) | | | | | 13.61 | 13.61 | 13.61 | 13.61 | 13.61 |
| pH (25°C, at 20-fold dilution) | | | | | 3.4 | 3.4 | 3.4 | 3.4 | 3.4 |
| Total amount of component (A) (mass%) | | | | | 0.353 | 0.353 | 0.353 | 0.353 | 0.353 |
| Total amount of component (B) (mass%) | | | | | 0.047 | 0.047 | 0.047 | 0.047 | 0.047 |
| Total amount of component (A) and component (B) (mass%) | | | | | 0.400 | 0.400 | 0.400 | 0.400 | 0.400 |
| Cation (A)/anion (B1)/anion (B2) charge ratio (molar ratio) | | | | | 55/36/9 | 55/36/9 | 55/36/9 | 55/36/9 | 55/36/9 |
| Mass ratio (A)/(B) | | | | | 7.55 | 7.55 | 7.55 | 7.55 | 7.55 |
| Content of component (B1) in component (B) (mass%) | | | | | 88.03 | 88.03 | 88.03 | 88.03 | 88.03 |
| Content of component (B2) in component (B) (mass%) | | | | | 11.97 | 11.97 | 11.97 | 11.97 | 11.97 |
| Mass ratio (B2)/{(B1) + (B2)} | | | | | 0.12 | 0.12 | 0.12 | 0.12 | 0.12 |
| Total amount of component (C) (mass%) | | | | | 0.40 | 0.40 | 0.40 | 0.40 | 0.40 |
| Mass ratio (C)/{(A) + (B)} | | | | | 1.00 | 1.00 | 1.00 | 1.00 | 1.00 |
| Evaluation results | Appearance and texture of hair conditioner after 2 days from blending | | | A: Aggregated particles are not visually recognized, and are slippery when crushed | A | A | A | A | A |
| | | | | A: Aggregated particles are not visually recognized, and are rough when crushed | | | | | |
| | | | | C: Aggregated particles are visually recognized | | | | | |
| | | | | D: Separated | | | | | |
| | Combing force after hair conditioner treatment + two times of hair washing | | | Standard: 1900 g or less *blank = 2055 g | 2005 | 1947 | 1962 | 1930 | 2004 |
| | Moisture resistance of hair after treatment | | | Standard: less than 1.3 | 1.53 | 1.40 | 1.42 | 1.37 | 1.33 |

The components listed in Tables 1 and 2 are as follows. The blending amount (mass%) described in the table is an effective amount of each component.
*1 Sodium alginate, "KIMICA ALGIN I-3-15" manufactured by Kimica Corporation
*2 Carboxyvinyl polymer, "Carbopol 980" manufactured by Lubrizol Advanced Materials, Inc.
*3 (Acrylates/alkyl acrylate (C10-30)) crosspolymer, "Pemulen TR-1" manufactured by Lubrizol Advanced Materials, Inc.
*4 (Acrylate/stearyl acrylate) copolymer, "SOFCARE SA-37W" manufactured by Kao Corporation, Mw: 20000
*5 Polyacrylic acid "Jurymer AC-10L" manufactured by TOAGOSEI CO., LTD., Mw: 20000 to 30000
*6 Polyethylene glycol, "BLAUNON PEG-20000S" manufactured by AOKI OIL INDUSTRIAL Co., Ltd., Mw: 20000
*7 Polyvinylpyrrolidone, "Luviskol K90" manufactured by BASF Japan Ltd., Mw: 1200000
*8 Polyoxyethylene polyoxypropylene glycol (150E.O.)(35P.O.), "PRONON #208" manufactured by NOF Corporation, Mw: 9000
*9 Polyoxyethylene polyoxypropylene glycol (240E.O.)(60P.O.), "Unilube 75DE-2620R" manufactured by NOF Corporation, Mw: 14000
*10 Dipropylene glycol, "DPG-RF" manufactured by ADEKA Corporation
*11 90% Lactic acid, "PURAC ULTRAPURE90" manufactured by PURAC Thailand Ltd.
*12 Polyquaternium-52 (N,N-dimethylaminoethyl methacrylate diethyl sulfate-N, N-dimethylacrylamide-polyethylene glycol dimethacrylate copolymer), "SOFCARE KG-101W-E" manufactured by Kao Corporation
*13 Polyquaternium-52 (N,N-dimethylaminoethyl methacrylate diethyl sulfate-N,N-dimethylacrylamide-polyethylene glycol dimethacrylate copolymer), "SOFCARE KG-301W" manufactured by Kao Corporation
*14 Polyquaternium-37 (methacryloylethyltrimethylammonium chloride polymer), "Cosmedia Ultragel 300" manufactured by BASF Japan Ltd., content of constituent unit represented by General Formula (1): 100% by mole

### Table 3

**Table 3**

| | | | | | Examples | | | | |
|---|---|---|---|---|---|---|---|---|---|
| | | | | | 12 | 13 | 14 | 15 | 16 |
| Step 1 | (B) | (B1) | Sodium alginate | KIMICA ALGIN I-3-150 (charge density 4.65 mmol/g) *1 (1% viscosity 300 to 400 mPa·s) | 0.041 | 0.041 | 0.041 | 0.041 | 0.041 |
| | | *(B2)* | *(Acrylic acid*/*stearyl acrylate) copolymer* | SOFCARE SA-37W * 2 (charge density 8.88mmol/e, Mw 20000) | *0.006* | *0.006* | *0.006* | *0.006* | *0.006* |
| | (C) | | Polyethylene glycol | BLAUNON PEG-20000S *3 | 0.40 | 0.40 | 0.40 | 0.40 | 0.40 |
| | (H) | | Dipropylene glycol | DPG-RF *4 | 2 | 2 | 2 | 2 | 2 |
| | Water | | Ion exchange water | | 74.68 | 1 | 5 | 10 | 20 |
| Step 2 | (D) | | Acid | 90% Lactic acid *5 | 0.315 | 0.315 | 0.315 | 0.315 | 0.315 |
| Step 3 | (A) | | Polyquaternium-52 | SOFCARE KG-101W-E (charge density 0.83 mmol/g) *6 (1% viscosity 2560 mPa·s) | 0.353 | 0.353 | 0.353 | 0.353 | 0.353 |
| Order of steps 1 to 3 | | | | | 1 → 2 → 3 | 1 → 2 → 3 | 1 → 2 → 3 | 1 → 2 → 3 | 1 → 2 → 3 |
| Concentration (mass%) of mixture 1 prepared in step 1 | | | | | 3.17 | 70.99 | 32.86 | 19.66 | 10.90 |
| Amount of water used in step 1 (mass% in composition as final product) | | | | | 74.73 | 1.05 | 5.05 | 10.05 | 20.05 |
| Amount of water used in step 2 (mass% in composition as final product) | | | | | 0.04 | 0.04 | 0.04 | 0.04 | 0.04 |
| *Amount of water used in step 3 (mass% in composition as final product)* | | | | | *13.61* | *13.61* | *13.61* | *13.61* | *13.61* |
| Amount of water in mixture obtained after step 3 | | | | | 88.38 | 14.70 | 18.70 | 23.70 | 33.70 |
| Step 4 | | | | | Step 4-2 | Step 4-1 | Step 4-1 | Step 4-1 | Step 4-1 |
| Oily phase used in step 4 | (E) | | 3ehenyltrimethylammonium chloride | QUARTAMIN 2285-E *7 | 1.2 | 1.2 | 1.2 | 1.2 | 1.2 |
| | (F) | | Cetanol | KALCOL 6870 *8 | 2.4 | 2.4 | 2.4 | 2.4 | 2.4 |
| | (G) | | Amino-modified silicone-dimethylpolysiloxane mixture | CF1046 *9 | 1.0 | 1.0 | 1.0 | 1.0 | 1.0 |
| | (H) | | Dipropylene glycol | DPG-RF *4 | 2 | 2 | 2 | 2 | 2 |
| | Other | | *Benzyl alcohol* | | *0.3* | *0.3* | *0.3* | *0.3* | *0.3* |
| Aqueous phase used in step 4 | (D) | | Lactic acid | 90% Lactic acid *5 | Appropriate amount | Appropriate amourt | Appropriate amount | Appropriate amount | Appropriate amount |
| | Water | | Ion exchange water | | Balance | Balance | Balance | Balance | Balance |
| pH (25°C, at 20-fold dilution) | | | | | 3.4 | 3.4 | 3.4 | 3.4 | 3.4 |
| Total amount of component (A) (mass%) | | | | | 0.353 | 0.353 | 0.353 | 0.353 | 0.353 |
| Total amount of component (B) (mass%) | | | | | 0.047 | 0.047 | 0.047 | 0.047 | 0.047 |
| Total amount of component (A) and component (B) (mass%) | | | | | 0.400 | 0.400 | 0.400 | 0.400 | 0.400 |
| Cation (A)/anion (B1)/anion (B2) charge ratio (molar ratio) | | | | | 55/36/9 | 55/36/9 | 55/36/9 | 55/36/9 | 55/36/9 |
| Mass ratio (A)/(B) | | | | | 7.55 | 7.55 | 7.55 | 7.55 | 7.55 |
| Content of component (B1) in component (B) (mass%) | | | | | 88.03 | 88.03 | 88.03 | 88.03 | 88.03 |
| Content of component (B2) in component (B) (mass%) | | | | | 11.97 | 11.97 | 11.97 | 11.97 | 11.97 |
| Mass ratio (B2)/{(B1) + (B2)} | | | | | 0.12 | 0.12 | 0.12 | 0.12 | 0.12 |
| Total amount of component (C) (mass%) | | | | | 0.40 | 0.40 | 0.40 | 0.40 | 0.40 |
| Mass ratio (C)/{(A) + (B)} | | | | | 1.00 | 1.00 | 1.00 | 1.00 | 1.00 |
| Evaluation results | Appearance and texture of hair conditioner after 2 days from blending | | | A: Aggregated particles are not visually recognized, and are slippery when crushed | A | C | A | A | A |
| | | | | A: Aggregated particles are not visually recognized, and are rough when crushed | | | | | |
| | | | | C: Aggregated particles are visually recognized | | | | | |
| | | | | D: Separated | | | | | |
| | Combing force after hair conditioner treatment + two times of hair washing | | | Standard: 1900 g or less *blank = 2055 g | 1333 | 1413 | 1563 | 1305 | 1538 |
| | Moisture resistance of hair after treatment | | | Standard: less than 1.3 | 1.19 | 1.11 | 1.15 | 1.12 | 1.10 |

The components listed in Table 3 are as follows. The blending amount (mass%) described in the table is an effective amount of each component.
*1 Sodium alginate, "KIMICA ALGIN I-3-15" manufactured by Kimica Corporation
*2 (Acrylate/stearyl acrylate) copolymer, "SOFCARE SA-37W" manufactured by Kao Corporation
*3 Polyethylene glycol, "BLAUNON PEG-20000S" manufactured by AOKI OIL INDUSTRIAL Co., Ltd., Mw: 20000
*4 Dipropylene glycol, "DPG-RF" manufactured by ADEKA Corporation
*5 90% Lactic acid, "PURAC ULTRAPURE90" manufactured from PURAC Thailand Ltd.
*6 Polyquaternium-52 (N,N-dimethylaminoethyl methacrylate diethyl sulfate-N,N-dimethylacrylamide-polyethylene glycol dimethacrylate copolymer), "SOFCARE KG-101W-E" manufactured by Kao Corporation
*7 Behenyltrimethylammonium chloride, "QUARTAMIN 2285-E" manufactured by Kao Corporation
*8 Cetanol, "Kalcol 6870" manufactured by Kao Corporation
*9 Amino-modified silicone-polydimethylsiloxane mixture "DOWSIL CF 1046" manufactured by Dow Toray Co., Ltd.

As shown in Tables 1 to 3, it can be seen that all of the hair conditioners which are aqueous compositions or emulsified compositions obtained by the production method of the present invention have high stability, and when used for hair treatment, the effect of suppressing entanglement of hair and the durability thereof can be improved, and spreading of hair after treatment under high humidity conditions can be suppressed.

In addition, according to the comparison of Examples 12 to 16 in Table 3, it is found that when the amount of water used in the step 1 is 5 mass% or more (Examples 13 to 16) in the production of the emulsified composition, the stability of the obtained emulsified composition is further improved.

### Industrial Applicability

The present invention can provide a method for producing an aqueous composition and an emulsified composition that contain a polyion complex, has high stability, can improve the texture improving effect of an object to be treated, the durability of the effect, and the moisture resistance, in particular, can improve the effect of suppressing entanglement of hair or fibers for head decoration products and the durability thereof when the compositions are used for treating hair or fibers for head decoration products, and can suppress the spread of hair or fibers for head decoration products after treatment under high humidity conditions.

## Claims

1. A method for producing an aqueous composition,
the aqueous composition comprising (A) a cationic polymer, (B) an anionic polymer, (C) a nonionic polymer, (D) an acid, and water,
the method comprising steps 1 to 3 described below in this order:
step 1: preparing a mixture 1 comprising the anionic polymer (B), the nonionic polymer (C), and water;
step 2: mixing the mixture 1 with the acid (D) or an aqueous solution thereof to prepare a mixture 2 comprising the anionic polymer (B), the nonionic polymer (C), the acid (D), and water; and
step 3: mixing the mixture 2 with the cationic polymer (A).

2. A method for producing an emulsified composition,
the emulsified composition comprising (A) a cationic polymer, (B) an anionic polymer, (C) a nonionic polymer, (D) an acid, an oily component, and water,
the method comprising steps 1 to 3 and step 4-1 or 4-2 described below in this order:
step 1: preparing a mixture 1 comprising the anionic polymer (B), the nonionic polymer (C), and water;
step 2: mixing the mixture 1 with the acid (D) or an aqueous solution thereof to prepare a mixture 2 comprising the anionic polymer (B), the nonionic polymer (C), the acid (D), and water;
step 3: mixing the mixture 2 with the cationic polymer (A) to prepare a mixture 3 comprising the cationic polymer (A), the anionic polymer (B), the nonionic polymer (C), the acid (D), and water;
step 4-1: mixing an aqueous phase containing water with an oily phase containing an oily component to prepare an emulsified product, and then mixing the emulsified product with the mixture 3; and
step 4-2: mixing the mixture 3 with an aqueous phase containing water, and then mixing the mixture with an oily phase containing an oily component for emulsification.

3. The method according to claim 2, wherein the oily component comprises (E) a cationic surfactant and (F) a higher alcohol.

4. The method according to any one of claims 1 to 3, wherein the component (B) comprises (B1) one or more selected from the group consisting of a cross-linked polymer containing a (meth)acrylic acid-derived constituent unit and an anionic polysaccharide.

5. The method according to claim 4, wherein the component (B) further contains (B2) an anionic polymer other than the component (B1).

6. The method according to any one of claims 1 to 5, wherein a mass ratio [(A)/(B)] of the component (A) to the component (B) contained in the composition is 1.0 or more and 30 or less.

7. The method according to claim 5 or 6, wherein a mass ratio [(B2)/{(B1) + (B2)}] of the component (B2) to a total amount of the component (B1) and the component (B2) is 0.01 or more and 1.0 or less.

8. The method according to any one of claims 1 to 7, wherein a total content of the component (A) and the component (B) contained in the composition is 0.02 mass% or more and 5.0 mass% or less.

9. The method according to any one of claims 1 to 8, wherein a mass ratio [(C)/{(A) + (B)}] of the component (C) to a total amount of the component (A) and the component (B) contained in the composition is 0.2 or more and 20 or less.
